# EUROPEAN PATENT SPECIFICATION

(11) **EP 3 601 325 B1**
(45) Date of publication and mention of the grant of the patent: **19.07.2023**
(21) Application number: 18715588.2
(22) Date of filing: 27.03.2018
(51) Int. Cl.: C07K 14/47, G01N 33/53, G01N 33/68

(54) **NEW TAU SPECIES**
NEUE ARTEN VON TAU
NOUVELLES ESPECES DE TAU

(30) Priority: 28.03.2017 EP 17305355
(43) Date of publication of application: 05.02.2020
(73) Proprietor: INSERM (Institut National de la Santé et de la Recherche Médicale), 75013 Paris (FR); Université de Lille, 59800 Lille (FR); Centre Hospitalier Regional Universitaire de Lille, 59000 Lille (FR); Centre National de la Recherche Scientifique (CNRS), 75016 Paris (FR); Ecole Supérieure de Physique et de Chimie Industrielles de la Ville de Paris, 75005 Paris (FR)
(72) Inventor: BUEE, Luc, 59045 Lille Cedex (FR); HAMDANE, Malika, 59045 Lille Cedex (FR); BLUM, David, 59045 Lille Cedex (FR); DERISBOURG, Maxime, 59045 Lille Cedex (FR); LEGHAY, Coline, 59045 Lille Cedex (FR); CHIAPPETTA, Giovanni, 75231 Paris Cedex 5 (FR); VINH, Joëlle, 75231 Paris Cedex 5 (FR); VERDIER, Yann, 75231 Paris Cedex 5 (FR)
(74) Representative: Inserm Transfert
(86) International application number: PCT/EP2018/057772
(87) International publication number: WO 2018/178078

(56) References cited:
- WO-A1-2011/056300
- MAXIME DERISBOURG ET AL: "Role of the Tau N-terminal region in microtubule stabilization revealed by new endogenous truncated forms", SCIENTIFIC REPORTS, vol. 5, no. 1, 14 May 2015 (2015-05-14), XP055395382, DOI: 10.1038/srep09659
- ROBERT A RISSMAN ET AL: "Caspase-cleavage of tau is an early event in Alzheimer disease tangle pathology", JOURNAL OF CLINICAL INVESTIGATION, AMERICAN SOCIETY FOR CLINICAL INVESTIGATION, US, vol. 114, no. 1, 1 July 2004 (2004-07-01), pages 121-130, XP002734348, ISSN: 0021-9738, DOI: 10.1172/JCI200420640
- TODD J. COHEN ET AL: "The acetylation of tau inhibits its function and promotes pathological tau aggregation", NATURE COMMUNICATIONS, vol. 2, 22 March 2011 (2011-03-22), page 252, XP055397255, DOI: 10.1038/ncomms1255
- HANNA TRZECIAKIEWICZ ET AL: "A Dual Pathogenic Mechanism Links Tau Acetylation to Sporadic Tauopathy", SCIENTIFIC REPORTS, vol. 7, 13 March 2017 (2017-03-13), page 44102, XP055397271, DOI: 10.1038/srep44102
- DAVID J. IRWIN ET AL: "Acetylated tau, a novel pathological signature in Alzheimer's disease and other tauopathies", BRAIN., vol. 135, no. 3, 24 February 2012 (2012-02-24), pages 807-818, XP055397395, GB ISSN: 0006-8950, DOI: 10.1093/brain/aws013
- MAR PEREZ ET AL: "Tau - an inhibitor of deacetylase HDAC6 function", JOURNAL OF NEUROCHEMISTRY, vol. 109, no. 6, 1 June 2009 (2009-06-01), pages 1756-1766, XP055044600, ISSN: 0022-3042, DOI: 10.1111/j.1471-4159.2009.06102.x
- SANG-WON MIN ET AL: "Critical role of acetylation in tau-mediated neurodegeneration and cognitive deficits", NATURE MEDICINE, vol. 21, no. 10, 21 September 2015 (2015-09-21), pages 1154-1162, XP055397314, ISSN: 1078-8956, DOI: 10.1038/nm.3951

## Description

### FIELD:

The disclosure relates to new Tau species starting from the methionine residue at position 11, said methionine being N-alpha acetylated, which have been isolated from human brain of an Alzheimer patient. The disclosure also relates to antibody that specifically binds this new tau species. These specific antibodies can be used for the diagnosis of Tauopathy disorders such as Alzheimer disease.

### BACKGROUND:

Alzheimer's disease (AD) is the most common dementia that firstly affects memory and cognitive functions and finally leads to care dependency and loss of autonomy. The number of the probability of developing dementia nearly doubles every five years. Alzheimer's Disease International association estimated that there are 46 million people affected with dementia worldwide in 2015, increasing to 131.5 million by 2050. AD has become a major public health issue. The total estimated worldwide costs of dementia are US$ 818 billion in 2015. Besides the economic aspect, AD is a real societal and human challenge to limit the consequences on our future generations.

The exact cause of the disease is actually not known and a delay in the diagnosis is damageable for the handling of patients in terms of optimal medical and social treatment. For decades, the amyloid cascade hypothesis has driven Alzheimer's research. However, in addition to the aggregation of the amyloid peptide into amyloid deposits, the other hallmark of the disease is the formation of neurofibrillary tangles made of the microtubule-associated Tau protein. In fact, Tau proteins are found aggregated and abnormally modified in degenerating neurons of AD patients. Progression of Tau pathology in cortical brain areas is closely correlated to cognitive impairment in AD. Currently, mechanisms leading to Tau pathology and its progression remain to be determined. Nevertheless, post-translational modifications of Tau proteins may be instrumental in the pathology. Thus, there is a continued need to develop novel diagnosis for tau disorders.

Tau proteins belong to the microtubule-associated proteins family and are found essentially in neurons where they are involved in the regulation of microtubule stability and dynamics as well as the axonal transport. There are six Tau isoforms in human adult brain with different amino-termini and containing in their carboxy-terminal part 4 microtubule binding domains (4R isoforms) or 3 microtubule binding domains (3R isoforms). Tau proteins are derived from a single gene by alternative splicing of exons 2, 3 and 10 (Sergeant et al., 2008). Tau proteins aggregate into filaments in a large group of neurodegenerative disorders referred to as Tauopathies. AD is the most common Tauopathy and form of dementia. One of the AD hallmarks is the neurofibrillary degeneration (NFD) that is composed of aggregated and abnormally phosphorylated Tau proteins. Both neuropathological and biochemical studies have previously shown that the progression of NFD in the cortical brain area is closely correlated to cognitive impairment in AD supporting a central role of Tau pathology (Braak and Braak, 1995; Duyckaert et al., 1998; Delacourte et al., 1999). Currently, mechanisms leading to NFD lesion and its progression remain to be determined. The main questions yet to be answered are what is the exact nature of the toxic Tau species and what are the mechanisms underlying their role in neurodegeneration. Among Tau species found in AD brains, truncated forms are of great interest and deserve to be more studied. Several yet unidentified truncated Tau fragments with broad range of molecular masses are found in AD brains suggesting therefore the occurrence of cleavages at both N-terminal and C-terminal parts of Tau proteins (Kovacech and Novak, 2010; Derisbourg et al., 2015). Besides, characterization of Tau in cerebrospinal fluid of AD and some related Tauopathies patients showed that it is mainly found as truncated species (Johnson et al., 1997, Barthelemy et al., 2016). A major step forward in the understanding of the relevance of Tau truncation in AD would be to identify the precise cleavage sites of Tau species in order to generate appropriate experimental tools. The formers are mandatory to investigate the impact of identified truncated-species on Tau function/dysfunction and its significance to AD pathology and its related Tauopathies. While several N- and C-terminally truncated Tau species are observed in human brains, only a limited number of specific Tau cleavage sites, after residues Asp13, Asp25, Asn368, Glu391 and Asp421, have been identified in situ. The species generated by these cleavages are found in neurofibrillary tangles, and their occurrence is correlated with the severity of AD disease (Novak et al., 1993; Gamblin et al., 2003; Guo et al., 2004 ; Horowitz et al., 2004 ; Rissman et al., 2004 ; Basurto-Islas et al., 2008 ; Zhang et al., 2014). Moreover, these species are able to reproduce Tau neurofibrillary degeneration in mice models (Zilka et al., 2006; de Calignon et al., 2010; McMillan et al., 2011) highlighting thereby the value of Tau truncation exploration to gain insight into mechanisms underlying Tau pathology.

In this context, inventors undertook a challenging proteomics approach to precisely identify new Tau cleavage sites, especially those at the N-terminus, which are less well characterized than those at the C-terminus. Inventors have identified several new N-terminally truncated Tau species in the human brain (Derisbourg et al., 2015). Among these new species, Tau fragment with Met11 as the N-terminal residue is of particular interest since this new site is located in the region encoded by exon1 that is shared by all Tau protein isoforms. Even though little is known about exon1 function, its modifications could impact on Tau function and pathology. Indeed, two missense mutations at arginine 5 residue (R5) have been reported as resulting in Tauopathies: R5H in a late-onset frontotemporal dementia subject (Hayashi et al., 2002) and R5L in a progressive supranuclear palsy subject (Poorkaj et al., 2002). Surprisingly, these mutations affect Tau fibrillation as well as its interaction with microtubules and axonal transport (Hayashi et al., 2002; Poorkaj et al., 2002; Magnani et al.; 2007). Given that microtubule binding domain (Goode et al., 1994; Gustke et al., 1994) as well as motifs involved in aggregation (von Bergen et al., 2000; Mukrasch et al., 2005) are located at c-terminal part of Tau protein, the role of N-terminus would be related to its involvement in the formation of a particular Tau structure. Indeed, Tau that is a natively unfolded protein likely adopts a "paperclip" conformation as a result of intra-molecular interaction between N-terminal and C-terminal domains (Jeganathan et al., 2006). Besides, epitope mapping studies of conformational antibodies that recognize selectively fibrillar Tau proteins show that both N-terminal and the microtubule binding domains are required for efficient antibodies binding supporting hence the idea of Tau folding on the basis of intermolecular interactions (Carmel et al., 1996; Jeganathan et al., 2008). Therefore, deletion of the outermost N-terminus of Tau, as encountered in Met11-Tau protein, would be of crucial functional and/or pathological consequences.

Regarding mechanisms leading to Met11-Tau fragment generation, the first thought would be to ascribe it to proteases that potentially could cleave Tau, such as caspases (for review, Mandelkow and Mandelkow 2012), but Met11 is not localized in canonical consensus environment for caspases cleavage. Interestingly, analysis of Tau mRNA sequence indicates that like start ATG (Met1), the ATG codon of Met11 is in an optimal context for translation initiation that matches with a minimal Kozak's consensus sequence (Kozak, 1987). Thereafter, one could ask whether Met11-Tau fragment is the product of an alternative translation site rather than a product of proteolysis cleavage. Alternative translation initiation at an internal initiation codon that produces N-terminal truncated variants has been described as a post-transcriptional mechanism of regulation of proteins function and metabololism (Courtois et al., 2002; Yin et al., 2002; Rocchi et al., 2013; Weingarten-Gabbay et al., 2013).

More interestingly, our further analysis showed that Met11-Tau is also detected as N-alpha-terminally acetylated form (unpublished data); such Tau species has never been described before. Tau N-terminal acetylation has been reported only for Alanine 2 (Hasegawa et al., 1992; Derisbourg et al., 2015).

N-terminal acetylation is a widespread post-translational modification that occurs in nearly 80% of mammalian proteins (Polevoda et al., 2003; Arnesen et al., 2009). Unlike reversible acetylation of ε-amino groups on lysine side chain by lysine acetyltransferases (KATs), N-teminal acetylation is irreversible and consists of transferring an acetyl group from acetyl coenzyme A to Nα group of the first amino acid residue of the substrate polypeptides by Nα-terminal acetyltransferases (NATs) (Drazic et al., 2016). This enzyme-catalyzed reaction occurs both at co-translational and post-translational level, commonly at Met residues (Driessen et al., 1985; Helsens et al., 2011,). N-terminal acetylation was mainly considered only as a regulator of proteins degradation; whether it acts as stabilizer or as degradation signal remains controversial. However, growing data indicate functional diversity of this post-translational modification, including regulation of protein-protein interactions, sub-cellular localization, membrane targeting and protein folding (for review, Aksnes et al., 2016). It is worthy to note that besides its role as microtubules associated protein, Tau exhibits other little known cellular localisations and functions. Tau was shown to interact with plasma membrane (Brandt et al., 1995), mitochondria (Jung et al., 1993) and with signaling molecules such as Src and Fyn tyrosine kinases (Lee et al., 1998; Burnouf et al., 2013). Tau could also be localized in the nucleus (Loomis et al., 1990; Sultan et al., 2011) and released outside cells (Frost et al., 2009; Dujardin et al., 2014). Hence, deciphering functional consequences of N-terminal acetylation could provide promising advance in our understanding of Tau biology and its related pathophysiological processes.

In eukaryotes, there are six different NATs (NatA-NatF) that are composed of a complex of catalytic and regulatory subunits and their substrate specificity depends on amino acid residue in the second position at protein N-terminus (Arnesen, 2011; Kalvik and Arnesen, 2013). Giving that NatB acetylates N-termini with acidic residue in the second position, Met11-Tau protein that have Glutamate residue at position 2 is thereafter a substrate candidate of the former. Moreover, a recent study has shown that NatB expression is regulated during brain development suggesting important roles, which remain to be uncovered, during neuronal differentiation and in post-mitotic neurons (Ohyama et al., 2012). Considering the importance of N-terminal acetylation, it is of great interest to explore it toward Tau protein metabolism and function and to establish the relevance of Ac-Met11-Tau species to AD and its related Taupathies.

Finally, there is a continued need to develop novel diagnosis for tau disorders.

### SUMMARY OF THE INVENTION:

The present invention is defined by the claims.

The invention provides an isolated Tau polypeptide which comprises at least 9 consecutive amino acids starting from the methionine residue at position 11 in SEQ ID NO:1-7 wherein said methionine at position 11 is N-alpha acetylated.

The invention further relates to an isolated polypeptide wherein the polypeptide is selected from the group consisting of:
(i) the amino acids sequence consisting of Tau N-Alpha Acetyl-Met11-352 (SEQ ID N 0:1);
(ii) the amino acids sequence consisting of Tau N-Alpha Acetyl-Met11-381 (SEQ ID N0:2);
(iii) the amino acids sequence consisting of Tau N-Alpha Acetyl-Met11-383 (SEQ ID N 0:3)
(iv) the amino acids sequence consisting of Tau N-Alpha Acetyl-Met11-410 (SEQ ID N 0:4)
(v) the amino acids sequence consisting of Tau N-Alpha Acetyl-Met11-412 ( SEQ ID N0:5);
(vi) the amino acids sequence consisting of Tau N-Alpha Acetyl-Met11-441 (SEQ ID N0:6);
(vii) the amino acids sequence consisting of Tau N-Alpha Acetyl-Met11-776 (SEQ ID N0:7);
(viii) an amino acid sequence at least 80% identical to the sequence of (i) to (vii), said sequence comprises the amino acid sequence starting from the N-Alpha Acetyl Methionine at position 11.
(ix) a fragment of at least 9 consecutive amino acids starting from the N-Alpha Acetyl methionine residue at position 11 of the sequence of (i), to (viii).

The invention further relates to an isolated polypeptide which comprises or consists of the following amino acid sequences (N-α-acetyl)MEDHAGTYGLG (SEQ ID NO:8).

The invention further relates to ab antibody that specifically binds to an isolated peptide of the invention wherein the antibody does not bind to a non N-alpha- acetylated form of Methionine 11 Tau polypeptide and/or a N-alpha-acetyl-Metl-Tau polypeptide.

The invention further relates to a kit comprising the antibody according to the invention.

The invention further relates to a method for detecting human Tau polypeptide according to the invention, and/or evaluating its amount in a biological sample.

The invention further relates to a diagnostic method of taupathies using the detection of the Tau polypeptide according to the invention.

The following detailed description, figures and example do not fall under the scope of the present disclosure and are present for understanding and illustration purposes only.

### DETAILED DESCRIPTION:

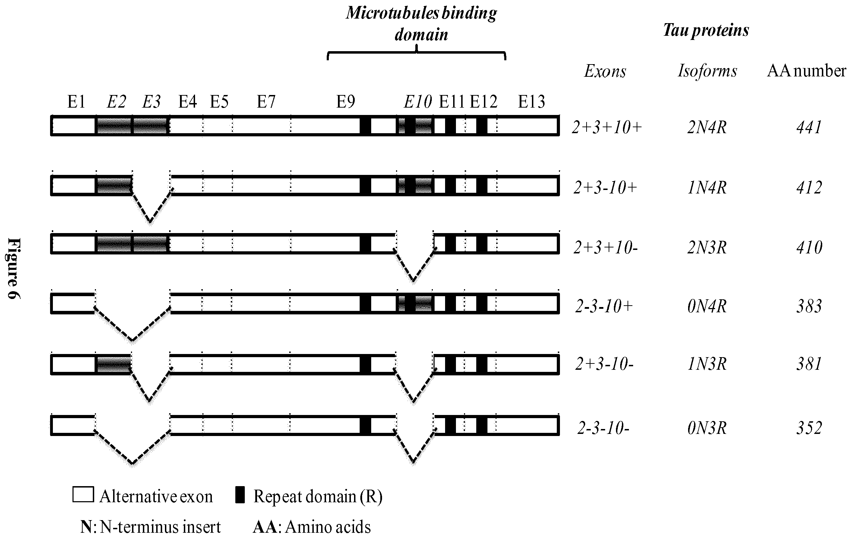

After using a proteomic approach, to identify new Tau species from the human brain (Derisbourg et al., 2015) inventors have subsequently identified a new Tau species starting at residue Met11 (Met11-Tau) which is N-alpha-terminally acetylated form (N-alpha-acetyl-Met11-Tau species: Ac-Met11-Tau). Such modification (N-alpha-acetyl-Met11) has never been described for Tau protein. Indeed N-alpha-acetyl-Met11 is located in the exon 1 of Tau protein, which is common to all Tau isoforms (see figure 6 of the several human Tau isoform). To evaluate the potential of this new Tau species as diagnostic biomarker, inventors have developed several monoclonal antibodies (such as 2H2/D11 antibody obtained from 2H2 hybridoma; see table 1) by using the first 11 amino acids of this new N-alpha-acetylated species as antigen. Such tools allow for the specific detection of the Ac-Met11-Tau species. After the validation of 2H2/D 11 specificity, this antibody was used to establish an association between Ac-Met11-Tau species and Tau pathology using the THY-Tau22 mouse model that develops with age neurofibrillary degeneration (NFD) and memory deficits (Schindowski et al., 2006; Van der Jeugd et al., 2013). Ac-Met11-Tau species were clearly detected in neurons displaying NFD on hippocampal brain sections. Interestingly, Ac-Met11-Tau species was detected in early stages of pathological process that precede memory deficits (as early as 2 months). Furthermore inventors have next examined sections of human brain hippocampus and immunohistochemistry analysis showed that 2H2D/11 antibody labels neurofibrillary tangles in AD brains while it is not reactive in hippocampus from elderly Controls. Finally, inventors developed an ELISA sandwich with 2H2/D11 which specifically recognized Ac-Met11-Tau species. By using such ELISA sandwich, AD brain samples are clearly discriminated from human elderly control brains.

### Isolated peptides

The invention disclosure relates to an isolated Tau polypeptide which comprises at least 9 consecutive amino acids starting from the methionine residue at position 11 in SEQ ID NO: 1-7 wherein said methionine at position 11 is N-alpha acetylated.

In particular, the isolated polypeptide of the invention comprises the amino acid sequence starting from the methionine at position 11 to the amino acid at position 776.

In the present disclosure the numbering of amino acids is according to the 441 aminoacids Tau isoform (SEQ ID NO: 6).

The invention also provides an isolated polypeptide selected from the group comprising or consisting of:
(i) the amino acids sequence consisting of Tau N-Alpha Acetyl-Met11-352 (SEQ ID N 0:1);
(ii) the amino acids sequence consisting of Tau N-Alpha Acetyl-Met11-381 ( SEQ ID N0:2);
(iii) the amino acids sequence consisting of Tau N-Alpha Acetyl-Met11-383 (SEQ ID N 0:3)
(iv) the amino acids sequence consisting of Tau N-Alpha Acetyl-Met11-410 (SEQ ID N 0:4)
(v) the amino acids sequence consisting of Tau N-Alpha Acetyl-Met11-412 ( SEQ ID N0:5);
(vi) the amino acids sequence consisting of Tau N-Alpha Acetyl-Met11-441 ( SEQ ID N0:6);
(vii) the amino acids sequence consisting of Tau N-Alpha Acetyl-Met11-776 (SEQ ID N0:7);
(viii) an amino acid sequence substantially homologous to the sequence of (i), to (vii) preferably an amino acid sequence at least 80% identical to the sequence of (i) to (vii)
(ix) a fragment of at least 9 consecutive amino acids starting from the N-Alpha Acetyl methionine residue at position 11 of the sequence of (i), to (viii).

In particular, the isolated Tau polypeptide comprises or consists of the amino acid sequence starting from the N-Alpha Acetyl Methionine at position 11 to the carboxy-terminal amino acids of the sequence of (i), to (viii).

As used herein, the term "amino acid" refers to natural or unnatural amino acids in their D and L stereoisomers for chiral amino acids. It is understood to refer to both amino acids and the corresponding amino acid residues, such as are present, for example, in peptidyl structure. Natural and unnatural amino acids are well known in the art. Common natural amino acids include, without limitation, alanine (Ala), arginine (Arg), asparagine (Asn), aspartic acid (Asp), cysteine (Cys), glutamine (Gln), glutamic acid (Glu), glycine (Gly), histidine (His), isoleucine (Ile), leucine (Leu), Lysine (Lys), methionine (Met), phenylalanine (Phe), proline (Pro), serine (Ser), threonine (Thr), tryptophan (Trp), tyrosine (Tyr), and valine (Val). Uncommon and unnatural amino acids include, without limitation, allyl glycine (AllylGly), norleucine, norvaline, biphenylalanine (Bip), citrulline (Cit), 4-guanidinophenylalanine (Phe(Gu)), homoarginine (hArg), homolysine (hLys), 2-naphtylalanine (2-Nal), ornithine (Orn) and pentafluorophenylalanine.

Amino acids are typically classified in one or more categories, including polar, hydrophobic, acidic, basic and aromatic, according to their side chains. Examples of polar amino acids include those having side chain functional groups such as hydroxyl, sulfhydryl, and amide, as well as the acidic and basic amino acids. Polar amino acids include, without limitation, asparagine, cysteine, glutamine, histidine, selenocysteine, serine, threonine, tryptophan and tyrosine. Examples of hydrophobic or non-polar amino acids include those residues having nonpolar aliphatic side chains, such as, without limitation, leucine, isoleucine, valine, glycine, alanine, proline, methionine and phenylalanine. Examples of basic amino acid residues include those having a basic side chain, such as an amino or guanidino group. Basic amino acid residues include, without limitation, arginine, homolysine and lysine. Examples of acidic amino acid residues include those having an acidic side chain functional group, such as a carboxy group. Acidic amino acid residues include, without limitation aspartic acid and glutamic acid. Aromatic amino acids include those having an aromatic side chain group. Examples of aromatic amino acids include, without limitation, biphenylalanine, histidine, 2-napthylalananine, pentafluorophenylalanine, phenylalanine, tryptophan and tyrosine. It is noted that some amino acids are classified in more than one group, for example, histidine, tryptophan and tyrosine are classified as both polar and aromatic amino acids. Amino acids may further be classified as non-charged, or charged (positively or negatively) amino acids. Examples of positively charged amino acids include without limitation lysine, arginine and histidine. Examples of negatively charged amino acids include without limitation glutamic acid and aspartic acid. Additional amino acids that are classified in each of the above groups are known to those of ordinary skill in the art.

The term "Tau" as used herein denotes the Tau protein from mammals and especially from primates (and Tupaiidae). Human Tau is a neuronal microtubule-associated protein found predominantly in axons and functions to promote tubulin polymerization and stabilize microtubules. Six isoforms (isoform A, B, C, D, E, F, G, fetal-Tau) are found in the human brain, the longest isoform comprising 441 amino acids (isoform F, Uniprot P10636-8). Tau and its properties are also described by Reynolds, C. H. et al., J. Neurochem. 69 (1997) 191-198. Tau, in its hyperphosphorylated form, is the major component of paired helical filaments (PHF), the building block of neurofibrillary lesions in Alzheimer's disease (AD) brain. Tau can be phosphorylated at its serine or threonine residues by several different kinases including GSK3beta, cdk5, MARK and members of the MAP kinase family.

The protein sequence of human Tau protein, and its isoforms, may be found in Uniprot database with the following access numbers:
Tau isoform Fetal (352 Amino Acids) Uniprot P10636-2
Tau isoform B (381 AA) Uniprot P10636-4
Tau isoform D (383 AA) Uniprot P10636-6
Tau isoform C (410 AA) Uniprot P10636-5
Tau isoform E (412 AA) Uniprot P10636-7
Tau isoform F (441AA) Uniprot P10636-8
Tau isoform G (776 AA) Uniprot P10636-9

A peptide "substantially homologous" to a reference peptide may derive from the reference sequence by one or more conservative substitutions. Two amino acid sequences are "substantially homologous" or "substantially similar" when one or more amino acid residue are replaced by a biologically similar residue or when greater than 80 % of the amino acids are identical, or greater than about 90 %, preferably greater than about 95%, are similar (functionally identical). Preferably, the similar, identical or homologous sequences are identified by alignment using, for example, the GCG (Genetics Computer Group, Program Manual for the GCG Package, Version 7, Madison, Wisconsin) pileup program, or any of the programs known in the art (BLAST, FASTA, etc.). The percentage of identity may be calculated by performing a pairwise global alignment based on the Needleman-Wunsch alignment algorithm to find the optimum alignment (including gaps) of two sequences along their entire length, for instance using Needle, and using the BLOSUM62 matrix with a gap opening penalty of 10 and a gap extension penalty of 0.5.

The term "conservative substitution" as used herein denotes the replacement of an amino acid residue by another, without altering the overall conformation and function of the peptide, including, but not limited to, replacement of an amino acid with one having similar properties (such as, for example, polarity, hydrogen bonding potential, acidic, basic, shape, hydrophobic, aromatic, and the like). Amino acids with similar properties are well known in the art. For example, arginine, histidine and lysine are hydrophilic-basic amino acids and may be interchangeable. Similarly, isoleucine, a hydrophobic amino acid, may be replaced with leucine, methionine or valine. Neutral hydrophilic amino acids, which can be substituted for one another, include asparagine, glutamine, serine and threonine.

By "substituted" or "modified" the present disclosure includes those amino acids that have been altered or modified from naturally occurring amino acids.

As such, it should be understood that in the context of the present disclosure, a conservative substitution is recognized in the art as a substitution of one amino acid for another amino acid that has similar properties.

According to the disclosure a first amino acid sequence having at least 80% of identity with a second amino acid sequence means that the first sequence has 80; 81; 82; 83; 84; 85; 86; 87; 88; 89; 90; 91; 92; 93; 94; 95; 96; 97; 98; or 99% of identity with the second amino acid sequence. Amino acid sequence identity is preferably determined using a suitable sequence alignment algorithm and default parameters, such as BLAST P (Karlin and Altschul, 1990).

In particular, the isolated peptide of the disclosure comprises at most 766 amino acids (and at least 9). In particular, the polypeptide of the disclosure comprises 766, 765, 764, 763, 762, 761, 760, 759, 758, 757, 756, 755, 754, 753, 752, 751, 750, 749, 748, 747, 746, 745, 744, 743, 742, 741, 740, 739, 738, 737, 736, 735, 734, 733, 732, 731, 730, 729, 728, 727, 726, 725, 724, 723, 722, 721, 720, 719, 718, 717, 716, 715, 714, 713, 712, 711, 710, 709, 708, 707, 706, 705, 704, 703, 702, 701, 700, 699, 698, 697, 696, 695, 694, 693, 692, 691, 690, 689, 688, 687, 686, 685, 684, 683, 682, 681, 680, 679, 678, 677, 676, 675, 674, 673, 672, 671, 670, 669, 668, 667, 666, 665, 664, 663, 662, 661, 660, 659, 658, 657, 656, 655, 654, 653, 652, 651, 650, 649, 648, 647, 646, 645, 644, 643, 642, 641, 640, 639, 638, 637, 636, 635, 634, 633, 632, 631, 630, 629, 628, 627, 626, 625, 624, 623, 622, 621, 620, 619, 618, 617, 616, 615, 614, 613, 612, 611, 610, 609, 608, 607, 606, 605, 604, 603, 602, 601, 600, 599, 598, 597, 596, 595, 594, 593, 592, 591, 590, 589, 588, 587, 586, 585, 584, 583, 582, 581, 580, 579, 578, 577, 576, 575, 574, 573, 572, 571, 570, 569, 568, 567, 566, 565, 564, 563, 562, 561, 560, ,559 558, 557, 556, 555, 554, 553, 552, 551, 550, 549, 548, 547, 546, 545, 544, 543, 542, 541, 540, 539, 538, 537, 536, 535, 534, 533, 532, 531, 530, 529, 528, 527, 526, 525, 524, 523, 522, 521, 520, 519, 518, 517, 516, 515, 514, 513, 512, 511, 510, 509, 508, 507, 506, 505, 504, 503, 502, 501, 500, 499, 498,497, 496, 495, 494, 493, 492, 491, 490, 489, 488, 487, 486, 485, 484, 483, 482, 481, 480, 479, 478, 477, 476, 475, 474, 473, 472, 471, 470, 469, 468, 467, 466, 465, 464, 463, 462, 461, 460, 459, 458, 457, 456, 455, 454, 453, 452, 451, 450, 449, 448, 447, 446, 445, 444, 443; 442 , 441, 440, 439, 438, 437 , 436, 435, 434, 433, 432, 431, 430, 429, 428, 427, 426, 425, 424, 423, 422, 421, 420, 419, 418, 417, 416, 415, 414, 413, 412, 411, 410, 409, 408, 407, 406, 405, 404, 403, 402, 401, 400, 399, 398, 397, 396, 395, 394, 393, 392, 391, 390, 389, 388, 387, 386, 385, 384, 383, 382, 381 , 380, 379, 378, 377, 376, 375, 374, 373, 372, 371, 370, 369, 368, 367, 366, 365, 364, 363, 362, 361, 360, 359, 358, 357, 356, 355, 354, 353, 352, 351, 350, 349, 348, 347, 346, 345, 344, 343, 342, 341, 340, 339, 338, 337, 336, 335, 334, 333, 332, 331, 330, 329, 328, 327, 326, 325, 324, 323, 322, 321, 320, 319, 318, 317, 316, 315, 314, 313, 312, 311, 310, 309, 308, 307, 306, 305, 304, 303, 302, 301, 300, 299, 298, 297, 296, 295, 294, 293, 292, 291, 290, 289, 288, 287, 286, 285, 284, 283, 282, ,281, 280, 279, 278, 277, 276, 275, 274, ,273 272, 271, 270, 269, 268, 267, 266, 265,264 263, 262, 261, 260, 259, 258, 257, 256, 255, 254, 253, ,252 , 251, 250, 249, 248, 247, 246, 245, 244, 243, 242, ,241, 240, 239, 238, 237, 236, 235, 234, 233, 232, 231, 230, 229, 228, 227, 226, 225, 224, 223, 222, 221, 220, 219, 218, 217, 216, 215, 214, 213, 212, 211, 210, 209, 208, 207, 206, 205, 204, 203, 202, 201, 200, 199, 198, 197, 196, 195, 194, 193, 192, 191, 190, 189, 188, 187, 186, 185, 184, 183, 182, 181, 180, 179, 178, 177, 176, 175, 174, 173, 172, 171, 170, 169, 168, 167, 166, 165, 164, 163, 162, 161, 160, 159, 158, 157, 156, 155, 154, 153, 152, 151, 150, 149, 148, 147, 146, 145, 144, 143, 142, 141, 140, 139, 138, 137, 136, 135, 134, 133, 132, 131, 130, 129, 128, 127, 126, 125, 124, 123, 122, 121, 120, 119, 118, 117, 116, 115, 114, 113, 112, 111, 110, 109, 108, 107, 106, 105, 104, 103, 102, 101, 100; 99; 98; 97; 96; 95; 94; 93; 92; 91; 90; 89; 88; 87; 86; 85; 84; 83; 82; 81; 80; 79; 78; 77; 76; 75; 74; 73; 72; 71; 70; 69; 68; 67; 66; 65; 64; 63; 62; 61; 60; 59; 58; 57; 56; 55; 54; 53; 52; 51; 50; 49; 48; 47; 46; 45; 44; 43; 42; 41; 40; 39; 38; 37; 36; 35; 34; 33; 32; 31; 30; 29; 28; 27; 26; 25; 24; 23; 22; 21; 20; 19; 18; 17; 16; 15; 14; 13; 12; 11; 10 or 9 amino acids. In particular, the polypeptide of the disclosure comprises less than 50 amino acids. In particular, the polypeptide of the disclosure comprises less than 30 amino acids. In particular, the polypeptide of the disclosure comprises less than 25 amino acids. In particular, the polypeptide of the disclosure comprises less than 20 amino acids. In particular, the polypeptide of the disclosure comprises less than 15 amino acids.

The isolated polypeptides according to the disclosure may be produced using any method known in the art. They may for example be produced as recombinant polypeptides in a host cell (e.g. in a bacterial, yeast or eukaryotic host cell), or chemically synthesized (see for review Kent S.B.H. Chem. Soc. Rev., 2009,38, 338-351 and Bradley L. et al Annu Rev Biophys Biomol Struct. 2005; 34: 91-118 or R. B. Merrifield (1969). "Solid-phase peptide synthesis." Advances in enzymology and related areas of molecular biology 32: 221-96.; R. B. Merrifield (1969). "The synthesis of biologically active peptides and proteins." JAMA 210(7): 1247-54. and Raibaut, L., O. El Mahdi and O. Melnyk (2015). "Solid Phase Protein Chemical Synthesis." Topics in current chemistry).

### Antibodies according to the disclosure

The inventors have generated specific antibodies directed against the polypeptide of the disclosure.

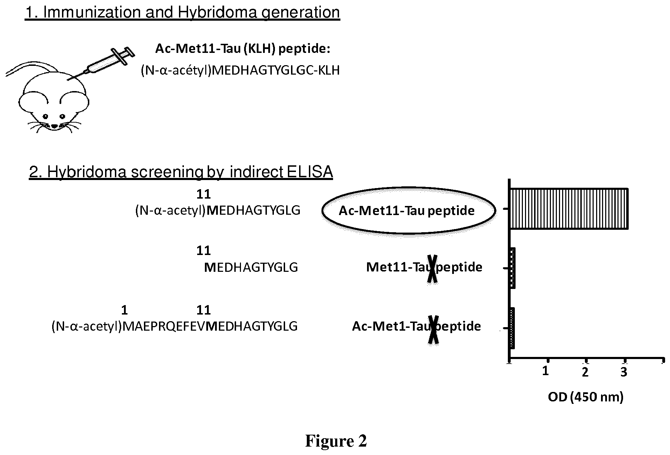

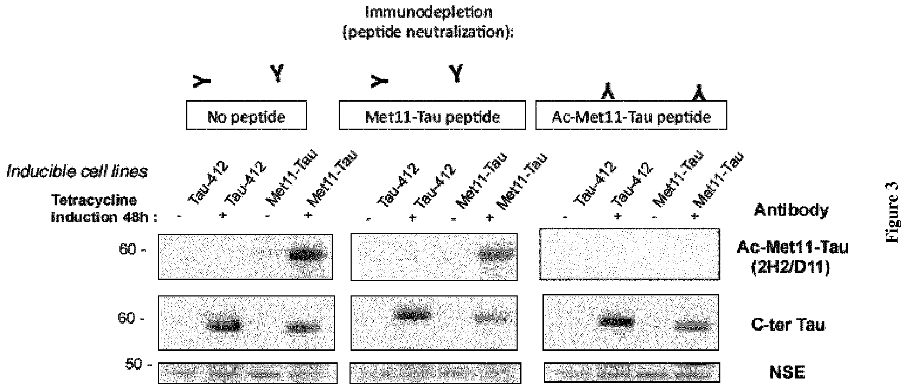

The monoclonal antibodies were produced by immunizing mice with the synthetic peptides, (N-α-acetyl)MEDHAGTYGLG (SEQ ID NO:8). More precisely, the inventors have found that antibodies screened for their capacity to recognize specifically the isolated polypeptide of the disclosure (figures 2 and 3) and to stain cell lines samples as well as brain samples from AD patients and from THY-Tau22 mouse model of tauopathies. Screening step of the antibodies of the disclosure has shown that these antibodies are specific of N-alpha acetylated form of Methionine 11 Tau species because they did not bind to the non-acetylated form of Methionine 11 Tau species nor to non-truncated Tau species, neither to the N-alpha-acetyl-Met1-Tau polypeptides (non-truncated Tau species)

The disclosure provides an antibody that specifically binds to an isolated polypeptide of the disclosure.

In particular, the antibody of the invention does not bind to a non N-alpha- acetylated form of Methionine 11 Tau polypeptide (i.e SEQ ID N°9) and/or a N-alpha-acetyl-Met1-Tau polypeptide (i.e SEQ ID N°10).

According to the present disclosure, "antibody" or "immunoglobulin" have the same meaning, and will be used equally in the present disclosure. The term "antibody" as used herein refers to immunoglobulin molecules and immunologically active portions of immunoglobulin molecules, i.e., molecules that contain an antigen binding site that immunospecifically binds an antigen. As such, the term antibody encompasses not only whole antibody molecules, but also antibody fragments as well as variants (including derivatives) of antibodies and antibody fragments. In natural antibodies, two heavy chains are linked to each other by disulfide bonds and each heavy chain is linked to a light chain by a disulfide bond. There are two types of light chain, lambda (l) and kappa (k). There are five main heavy chain classes (or isotypes) which determine the functional activity of an antibody molecule: IgM, IgD, IgG, IgA and IgE. Each chain contains distinct sequence domains. The light chain includes two domains, a variable domain (VL) and a constant domain (CL). The heavy chain includes four domains, a variable domain (VH) and three constant domains (CH1, CH2 and CH3, collectively referred to as CH). The variable regions of both light (VL) and heavy (VH) chains determine binding recognition and specificity to the antigen. The constant region domains of the light (CL) and heavy (CH) chains confer important biological properties such as antibody chain association, secretion, trans-placental mobility, complement binding, and binding to Fc receptors (FcR). The Fv fragment is the N-terminal part of the Fab fragment of an immunoglobulin and consists of the variable portions of one light chain and one heavy chain. The specificity of the antibody resides in the structural complementarity between the antibody combining site and the antigenic determinant. Antibody combining sites are made up of residues that are primarily from the hypervariable or complementarity determining regions (CDRs). Occasionally, residues from nonhypervariable or framework regions (FR) influence the overall domain structure and hence the combining site. Complementarity Determining Regions or CDRs refer to amino acid sequences which together define the binding affinity and specificity of the natural Fv region of a native immunoglobulin binding site. The light and heavy chains of an immunoglobulin each have three CDRs, designated VL-CDR1, VL-CDR2, VL-CDR3 and VH-CDR1, VH-CDR2, VH-CDR3, respectively. An antigen-binding site, therefore, includes six CDRs, comprising the CDR set from each of a heavy and a light chain V region. Framework Regions (FRs) refer to amino acid sequences interposed between CDRs.

Antibody binding to isolated polypeptide of the disclosure can be assayed by conventional methods known in the art. The mature form of polypeptide of the disclosure is preferably used for assaying antibody binding to epitope of polypeptide of the disclosure. Alternatively, any variant form of isolated polypeptide of the disclosure that retains binding of mAb 2H2/D11 can be used. Many different competitive binding assay format(s) can be used for determining epitope binding. The immunoassays which can be used include, but are not limited to, competitive assay systems using techniques such as radioimmunoassays, ELISA, "sandwich" immunoassays, immunoprecipitation assays, fluorescent immunoassays, protein A immunoassays, and complement-fixation assays. Such assays are routine and well known in the art (see, e.g., Ausubel et al., eds, 1994 Current Protocols in Molecular Biology, Vol. 1, John Wiley & sons, Inc., New York). For example, the BIACORE^{®} (GE Healthcare, Piscaataway, NJ) is one of a variety of surface plasmon resonance assay formats that are routinely used to epitope bin panels of monoclonal antibodies. Additionally, routine cross-blocking assays such as those described in Antibodies, A Laboratory Manual, Cold Spring Harbor Laboratory, Ed Harlow and David Lane, 1988, can be performed. An example of a suitable ELISA assay is also described in the Example below.

As used herein, the term "Affinity" refers to the strength of interaction between antibody and antigen at single antigenic sites. Within each antigenic site, the variable region of the antibody "arm" interacts through weak non-covalent forces with the antigen at numerous sites; the more interactions, the stronger the affinity. Affinity can be determined by measuring K_{D}. The term "K_{D}", as used herein, is intended to refer to the dissociation constant, which is obtained from the ratio of K_{d} to Kₐ (i.e. K_{d}/Kₐ) and is expressed as a molar concentration (M). K_{D} values for antibodies can be determined using methods well established in the art. A method for determining the K_{D} of an antibody is by using surface plasmon resonance, or using a biosensor system such as a Biacore^{®} system.

The disclosure provides an antibody that specifically binds the amino acids sequence consisting of (N-α-acetyl)MEDHAGTYGLG (SEQ ID NO:8)

The disclosure further provides an antibody that specifically binds to an isolated polypeptide comprising or consisting of:
i) the amino acids sequence consisting of Tau N-Alpha Acetyl-Met11-352 (SEQ ID N 0:1);
ii) the amino acids sequence consisting of Tau N-Alpha Acetyl-Met11-381 ( SEQ ID N0:2);
iii) the amino acids sequence consisting of Tau N-Alpha Acetyl-Met11-383 (SEQ ID N 0:3)
iv) the amino acids sequence consisting of Tau N-Alpha Acetyl-Met11-410 (SEQ ID N 0:4)
v) the amino acids sequence consisting of Tau N-Alpha Acetyl-Met11-412 ( SEQ ID N0:5);
vi) the amino acids sequence consisting of Tau N-Alpha Acetyl-Met11-441 ( SEQ ID N0:6);
vii) the amino acids sequence consisting of Tau N-Alpha Acetyl-Met11-776 (SEQ ID N0:7);
viii) an amino acid sequence substantially homologous to the sequence of (i), to (vii) preferably an amino acid sequence at least 80% identical to the sequence of (i) to (vii)
ix) a fragment of at least 9 consecutive amino acids starting from the N-Alpha Acetyl methionine residue at position 11 of the sequence of (i), to (viii).

These antibodies can recognize an epitope located within, or comprising at least one amino acid located within, the fragment of at least 9 consecutive amino acids starting from the methionine residue at position 11 of any one of isolated polypeptide (i) to (viii).

Preferably, said epitope is located within the fragment comprising or consisting of any one of isolated polypeptide (i) to (viii).

Most preferably said epitope is located within the peptide (N-α-acetyl)MEDHAGTYGLG (SEQ ID NO:8). Such antibodies are characterized in that they specifically bind to N-alpha-acetyl-Met11-Tau species of the disclosure.

These antibodies can be polyclonal or monoclonal. When the antibodies are monoclonal, they can for example correspond to chimeric, humanized or fully human antibodies, antibody fragment and single domain antibody.

The term "chimeric antibody" refers to an antibody which comprises a VH domain and a VL domain of an antibody, and a CH domain and a CL domain of a human antibody.

According to the disclosure, the term "humanized antibody" refers to an antibody having variable region framework and constant regions from a human antibody but retains the CDRs of a previous non human antibody.

The term "antibody fragment" refers to a fragment of an antibody which contain the variable domains comprising the CDRs of said antibody. The basic antibody fragments include Fab, Fab', F(ab')2 Fv, scFv, dsFv. For example of antibody fragment see also for review, Holliger et al Nature Biotechnology 23, issue 9 1126 - 1136 (2005), which are includes herein by reference.

The term "Fab" denotes an antibody fragment having a molecular weight of about 50,000 and antigen binding activity, in which about a half of the N-terminal side of H chain and the entire L chain, among fragments obtained by treating IgG with a protease, papaine, are bound together through a disulfide bond.

The term "F(ab')2" refers to an antibody fragment having a molecular weight of about 100,000 and antigen binding activity, which is slightly larger than the Fab bound via a disulfide bond of the hinge region, among fragments obtained by treating IgG with a protease, pepsin.

The term "Fab' " refers to an antibody fragment having a molecular weight of about 50,000 and antigen binding activity, which is obtained by cutting a disulfide bond of the hinge region of the F(ab')2.

A single chain Fv ("scFv") polypeptide is a covalently linked VH::VL heterodimer which is usually expressed from a gene fusion including VH and VL encoding genes linked by a peptide-encoding linker. "dsFv" is a VH::VL heterodimer stabilised by a disulfide bond. Divalent and multivalent antibody fragments can form either spontaneously by association of monovalent scFvs, or can be generated by coupling monovalent scFvs by a peptide linker, such as divalent sc(Fv)2.

The term "diabodies" "tribodies" or "tetrabodies" refers to small antibody fragments with multivalent antigen-binding sites (2, 3 or four), which fragments comprise a heavy-chain variable domain (VH) connected to a light-chain variable domain (VL) in the same polypeptide chain (VH-VL). By using a linker that is too short to allow pairing between the two domains on the same chain, the domains are forced to pair with the complementary domains of another chain and create two antigen-binding sites.

As used herein the term "single domain antibody" has its general meaning in the art and refers to the single heavy chain variable domain of antibodies of the type that can be found in Camelid mammals which are naturally devoid of light chains. Such single domain antibody are also called VHH or "nanobody^{®}". For a general description of (single) domain antibodies, reference is also made to the prior art cited above, as well as to EP 0 368 684, Ward et al. (Nature 1989 Oct 12; 341 (6242): 544-6), Holt et al., Trends Biotechnol., 2003, 21(11):484-490; and WO 06/030220, WO 06/003388. The nanobody has a molecular weight approximately one-tenth that of a human IgG molecule, and the protein has a physical diameter of only a few nanometers. One consequence of the small size is the ability of camelid nanobodies to bind to antigenic sites that are functionally invisible to larger antibody proteins, i.e. , camelid nanobodies are useful as reagents detect antigens that are otherwise cryptic using classical immunological techniques, and as possible therapeutic agents. Thus yet another consequence of small size is that a nanobody can inhibit as a result of binding to a specific site in a groove or narrow cleft of a target protein, and hence can serve in a capacity that more closely resembles the function of a classical low molecular weight drug than that of a classical antibody. The low molecular weight and compact size further result in nanobodies being extremely thermostable, stable to extreme pH and to proteolytic digestion, and poorly antigenic. Another consequence is that nanobodies readily move from the circulatory system into tissues, and even cross the blood-brain barrier and can treat disorders that affect nervous tissue. Nanobodies can further facilitated drug transport across the blood brain barrier. See U.S. patent application 20040161738 published August 19, 2004. These features combined with the low antigenicity to humans indicate great therapeutic potential. The amino acid sequence and structure of a single domain antibody can be considered to be comprised of four framework regions or "FRs" which are referred to in the art and herein as "Framework region 1" or "FRl "; as "Framework region 2" or "FR2"; as "Framework region 3 " or "FR3"; and as "Framework region 4" or "FR4" respectively; which framework regions are interrupted by three complementary determining regions or "CDRs", which are referred to in the art as "Complementarity Determining Region for "CDR1"; as "Complementarity Determining Region 2" or "CDR2" and as "Complementarity Determining Region 3" or "CDR3", respectively. Accordingly, the single domain antibody can be defined as an amino acid sequence with the general structure : FRl - CDR1 - FR2 - CDR2 - FR3 - CDR3 - FR4 in which FRl to FR4 refer to framework regions 1 to 4 respectively, and in which CDRl to CDR3 refer to the complementarity determining regions 1 to 3. In the context of the disclosure, the amino acid residues of the single domain antibody are numbered according to the general numbering for VH domains given by the International ImMunoGeneTics information system aminoacid numbering (http://imgt.cines.fr/).

Methods for obtaining such antibodies are well known in the art. For example, monoclonal antibodies according to the disclosure can be obtained through immunization of a non-human mammal with said fragment comprising or consisting of any one of (i) to (vii). Starting from the polyclonal antibodies, one can then obtain monoclonal antibodies using standard methods.

An antibody of the disclosure can be conjugated with a detectable label to form an immunoconjugate. Suitable detectable labels include, for example, a radioisotope, a fluorescent label, a chemiluminescent label, an enzyme label, a bioluminescent label or colloidal gold. Methods of making and detecting such detectably-labeled immunoconjugates are well-known to those of ordinary skill in the art, and are described in more detail below.

The detectable label can be a radioisotope that is detected by autoradiography. Isotopes that are particularly useful for the purpose of the present disclosure are ³H, ¹²⁵I, ¹³¹I, ³⁵S and ¹⁴C.

Immunoconjugates can also be labeled with a fluorescent compound. The presence of a fluorescently-labeled antibody is determined by exposing the immunoconjugate to light of the proper wavelength and detecting the resultant fluorescence. Fluorescent labeling compounds include fluorescein isothiocyanate, rhodamine, phycoerytherin, phycocyanin, allophycocyanin, o-phthaldehyde and fluorescamine.

Alternatively, immunoconjugates can be detectably labeled by coupling an antibody to a chemiluminescent compound. The presence of the chemiluminescent-tagged immunoconjugate is determined by detecting the presence of luminescence that arises during the course of a chemical reaction. Examples of chemiluminescent labeling compounds include luminol, isoluminol, an aromatic acridinium ester, an imidazole, an acridinium salt and an oxalate ester.

Similarly, a bioluminescent compound can be used to label immunoconjugates of the present disclosure. Bioluminescence is a type of chemiluminescence found in biological systems in which a catalytic protein increases the efficiency of the chemiluminescent reaction. The presence of a bioluminescent protein is determined by detecting the presence of luminescence. Bioluminescent compounds that are useful for labeling include luciferin, luciferase and aequorin.

Alternatively, immunoconjugates can be detectably labeled by linking a monoclonal antibody to an enzyme. When the enzyme conjugate is incubated in the presence of the appropriate substrate, the enzyme moiety reacts with the substrate to produce a chemical moiety which can be detected, for example, by spectrophotometric, fluorometric or visual means. Examples of enzymes that can be used to detectably label polyspecific immunoconjugates include β-galactosidase, glucose oxidase, peroxidase and alkaline phosphatase.

An antibody of the disclosure may be labelled with a metallic chemical element such as lanthanides. Lanthanides offer several advantages over other labels in that they are stable isotopes, there are a large number of them available, up to 100 or more distinct labels, they are relatively stable, and they are highly detectable and easily resolved between detection channels when detected using mass spectrometry. Lanthanide labels also offer a wide dynamic range of detection. Lanthanides exhibit high sensitivity, are insensitive to light and time, and are therefore very flexible and robust and can be utilized in numerous different settings. Lanthanides are a series of fifteen metallic chemical elements with atomic numbers 57-71. They are also referred to as rare earth elements. Lanthanides may be detected using CyTOF technology. CyTOF is inductively coupled plasma time-of-flight mass spectrometry (ICP-MS). CyTOF instruments are capable of analyzing up to 1000 cells per second for as many parameters as there are available stable isotope tags.

Those of skill in the art will know of other suitable labels which can be employed in accordance with the present disclosure. The binding of marker moieties to monoclonal antibodies can be accomplished using standard techniques known to the art.

Moreover, the convenience and versatility of immunochemical detection can be enhanced by using monoclonal antibodies that have been conjugated with avidin, streptavidin, and biotin.

### Detectins and Diagnostic methods of the disclosure:

An object of the invention is a method for detecting human N-alpha-acetyl-Met11-Tau species of the invention, and/or evaluating its amount in a biological sample.

Biological sample, means without limitation a culture medium and cell samples, a whole blood sample, a serum sample, a plasma sample, an urinary sample, a salivary sample, a cerebrospinal fluid sample, or a brain tissue sample.

Detecting the N-alpha-acetyl-Met11-Tau species may include separation of the proteins/polypeptides: centrifugation based on the protein's molecular weight; electrophoresis based on mass and charge; HPLC based on hydrophobicity; size exclusion chromatography based on size; and solid-phase affinity based on the protein's affinity for the particular solid-phase that is use. Once separated, N-alpha-acetyl-Met11-Tau species may be identified based on the known "separation profile" e. g., retention time, for that protein and measured using standard techniques. Alternatively, the separated proteins may be detected and measured by, for example, a mass spectrometer (see example section).

The detection and amount of the N-alpha-acetyl-Met11-Tau species of the disclosure may be determined by using standard electrophoretic and immunodiagnostic techniques, including immunoassays such as competition, direct reaction such as immunohistochemistry, or sandwich type assays. Such assays include, but are not limited to, Western blots; agglutination tests; enzyme-labelled and mediated immunoassays, such as ELISAs; biotin/avidin type assays; radioimmunoassays; immunoelectrophoresis; immunoprecipitation, etc. The reactions generally include revealing labels such as fluorescent, chemiluminescent, radioactive, enzymatic labels or dye molecules, or other methods for detecting the formation of a complex between the antigen and the antibody or antibodies reacted therewith.

For example, determination of the N-alpha-acetyl-Met11-Tau species amount can be performed by a variety of techniques and method any well known method in the art: RIA kits (DiaSorin; IDS, Diasource) Elisa kits (Fujirebio, Thermo Fisher, EHTGFBI, R&D DY2935, IDS (manual) IDS (adapted on open analyzers) Immunochemiluminescent automated methods (MesoScaleDiscovery, DiaSorin Liaison, Roche Elecsys family, IDS iSYS) (Janssen et al., 2012) Simoa/Quanterix.

In particular, the methods of the disclosure comprise contacting the biological sample with a binding partner.

As used therein, binding partner refers to a molecule capable of selectively interacting with N-alpha-acetyl-Met11-Tau species of the disclosure.

The binding partner may be generally an antibody that may be polyclonal or monoclonal, preferably monoclonal.

In particular, the binding partner may be an aptamer. Aptamers are a class of molecule that represents an alternative to antibodies in term of molecular recognition. Aptamers are oligonucleotide or oligopeptide sequences with the capacity to recognize virtually any class of target molecules with high affinity and specificity. Such ligands may be isolated through Systematic Evolution of Ligands by EXponential enrichment (SELEX) of a random sequence library, as described in Tuerk et al. (1990) Science, 249, 505-510. The random sequence library is obtainable by combinatorial chemical synthesis of DNA. In this library, each member is a linear oligomer, eventually chemically modified, of a unique sequence. Possible modifications, uses and advantages of this class of molecules have been reviewed in Jayasena 1999. Peptide aptamers consist of conformationally constrained antibody variable regions displayed by a platform protein, such as E. coli Thioredoxin A, that are selected from combinatorial libraries by two hybrid methods (Colas et al. (1996) Nature, 380, 548-50).

The binding partners of the disclosure such as antibodies or aptamers, may be labelled with a detectable molecule or substance, such as a fluorescent molecule, a radioactive molecule or any others labels known in the art. Labels are known in the art that generally provide (either directly or indirectly) a signal.

As used herein, the term "labelled", with regard to the binding partner, is intended to encompass direct labelling of the antibody or aptamer by coupling (i.e., physically linking) a detectable substance, such as a radioactive agent or a fluorophore (e.g. fluorescein isothiocyanate (FITC) or phycoerythrin (PE) or Indocyanine (Cy5) to the antibody or aptamer, as well as indirect labelling of the probe or antibody by reactivity with a detectable substance. An antibody or aptamer of the disclosure may be labelled with a radioactive molecule by any method known in the art. For example radioactive molecules include but are not limited radioactive atom for scintigraphic studies such as I123, I124, In111, Re186, Re188.

The aforementioned assays generally involve the bounding of the binding partner (ie. antibody or aptamer) in a solid support. Solid supports which can be used in the practice of the disclosure include substrates such as nitrocellulose (e. g., in membrane or microtiter well form); polyvinylchloride (e. g., sheets or microtiter wells); polystyrene latex (e.g., beads or microtiter plates); polyvinylidine fluoride; diazotized paper; nylon membranes; activated beads, magnetically responsive beads, and the like. More particularly, an ELISA method can be used, wherein the wells of a microtiter plate are coated with a set of antibodies against N-alpha-acetyl-Met11-Tau species. A body fluid sample containing or suspected of containing N-alpha-acetyl-Met11-Tau species is then added to the coated wells. After a period of incubation sufficient to allow the formation of binding partner- N-alpha-acetyl-Met11-Tau species complexes, the plate(s) can be washed to remove unbound material and a labelled secondary binding molecule added. The secondary binding molecule is allowed to react with any captured sample marker protein, the plate washed and the presence of the secondary binding molecule detected using methods well known in the art.

As the binding partner, the secondary binding molecule may be labelled.

Antibodies of the present disclosure and immunoconjugates can be used for detecting N-alpha-acetyl-Met11-Tau species, and/or evaluating its amount in a biological sample, in particular a culture medium and cell samples, a whole blood sample, a serum sample, a plasma sample, a cerebrospinal fluid sample, or a brain tissue sample. Therefore they can be used for diagnosing all tau diseases.

Accordingly, the method of detection of the invention is consequently useful for the *in vitro* diagnosis of tauopathies from a biological sample. In particular, the method of detection of the disclosure is consequently useful for the *in vitro* diagnosis of early stage tauopathy from a biological sample.

As used herein, the term "early stage taupathies" refers to the stage of the disease before the onset of clinical symptoms, such as memory loss.

As used herein, the term "biological sample" as used herein refers to any biological sample of a subject. Preferably, said sample is a body fluid of said subject. Non-limiting examples of samples include, but are not limited to, blood, serum, plasma, urine, saliva, and cephalorachidian liquid (CRL).

A further object of the invention is a method for detecting N-alpha-acetyl-Met11-Tau species, and/or evaluating its amount in a biological sample, wherein said method comprises contacting said sample with an antibody of the invention under conditions allowing the formation of an immune complex between N-alpha-acetyl-Met11-Tau species and said antibody/immunoconjugate, and detecting or measuring the immune complex formed.

The immune complex formed can be detected or measured by a variety of methods using standard techniques, including, by way of non-limitative examples, enzyme-linked immunosorbent assay (ELISA) or other solid phase immunoassays, radioimmunoassay, electrophoresis, immunofluorescence, or Western blot.

A further object of the disclosure is a method for in vitro diagnosing a tauopathy, wherein said method comprising detecting the presence of N-alpha-acetyl-Met11-Tau species, as indicated above, in a biological sample from a subject to be tested.

The term "tauopathy" has its general meaning in the art and refers to a disease characterized by Tau aggregation (Iqbal, K. et al. Biochimica et Biophysica Acta (BBA) 1739 (2005) 198-210). Tauopathies include among others, Alzheimer's Disease, Down syndrome; Guam parkinsonism dementia complex; Dementia pugilistica and other chronic traumatic encephalopathies; myotonic dystrophies; Niemann-Pick disease type C; Pick disease; argyrophilic grain disease; Fronto-temporal dementia; Cortico-basal degeneration; Pallido-ponto-nigral degeneration; Progressive supranuclear palsy; and Prion disorders such as Gerstmann-Sträussler-Scheinker disease with tangles.

Finally, the invention also provides kits comprising at least one antibody of the invention or a fragment thereof. Kits of the disclosure can contain an antibody coupled to a solid support, e.g., a tissue culture plate or beads (e.g., sepharose beads). Kits can be provided which contain antibodies for detection and quantification of N-alpha-acetyl-Met11-Tau species in vitro, e.g. in an ELISA or a Western blot. Such antibody useful for detection may be provided with a label such as a fluorescent or radiolabel.

### FIGURES:

**Figure 1****. MS/MS spectra of N-terminally acetylated peptide Met11-Tau from human brain.** MS/MS spectra of N-terminally acetylated peptide Met11-Tau from human brain (Tau pathology, Braak III). Charge:+2. Monoisotopic m/z: 732.31 Da; MH+: 1463.62.RT: 27.86. Scan 154. G140418_0427_c_ymv.raw Identified with Mascot v1.30; ion score = 54; exp value: 3E-003
**Figure 2****. Generation of specific monoclonal antibody targeting Ac-Met11-Tau secreted by 2H2/D11 hybridoma.** Schematic representation of 2H2/D11 antibody production. Amino acids of the peptide used for mice immunization and of the peptides used for Hyibridoma supernatants screening are shown. Histograms indicated representative ELISA OD values obtained by 2H2/D11 hybridoma supernatant.
**Figure 3****. Validation of 2H2/D11 antibody specificity by Western Blot and peptide immunodepletion experiments.**
**Figure 4** **2H2/D11 antibody allows specific detection of N-terminally acetylated Met11-Tau protein by sandwich ELISA.**
**Figure 5****. 2H2/D11 antibody based sandwich ELISA in AD and control brain samples (Temporal cortex)**
**Figure 6****. A. The 6 Human Tau isoform in Brain.**
**Figure 7****. 2H2/D11 antibody allows specific detection of N-alpha-terminally acetylated Met11-Tau peptide by indirect ELISA.**
**Figure 8****. Specific detection of Ac-Met11-Tau in AD hippocampus, by Sandwich ELISA.**

### EXAMPLE 1:

### Materials and Methods

Human tissue samples. Human brain autopsy samples were from the Lille NeuroBank collection (Centre de Ressources Biologiques du CHU de Lille). Informed consent was obtained from all subjects. The Lille NeuroBank has been declared to the French Research Ministry by the Lille University Hospital (CHU-Lille) on August 14, 2008 under the reference DC-2000-642 and fulfills the criteria defined by French Law regarding biological resources, including informed consent, ethics review committee approval and data protection. The study was approved by the ethics review committee of Lille NeuroBank. The stages of Tau pathology were categorized on the basis of neuropathological characteristics according to Braak and Braak (2011).

Identification of Ac-Met11-Tau species. Ac-Met11-Tau was identified using the experimental approaches detailed in Derisbourg et al (2015). Briefly, Tau species were enriched by immunoprecipitation from human occipital cortex brain, labeled with biotin and analyzed by capillary liquid chromatography-tandem mass spectrometry (LC-MS/MS).

Generation of 2H2/D11 antibody. 2H2/D11 antibody was generated by immunization with an N-alpha-terminal acetyl Tau peptide (Ac-Met11-Tau peptide: {Nα-acetyl}MEDHAGTYGLG : SEQ ID N°8) corresponding to the Tau sequence from Methionine at position 11 to Glycine at position 21. This sequence is encoded by exon1; shared by all Tau isoforms. A cysteine residue has been added to the C-terminus of Ac-Met11-Tau peptide for KLH Conjugation. Balb/c mice were immunized subcutaneously with 3 boosts at days 14, 45 and 63. The lymphocytes from the spleen of the mouse displaying the highest titer were then fused with NS1 myeloma cells, according to the method described in (Pandey, 2010). The hybridoma supernatants were screened in indirect ELISA against the following different peptides:
Ac-Met11-Tau peptide: {Nα-acetyl}MEDHAGTYGLG; (SEQ ID N°8) the same sequence than Tau fragment used as antigen.
Met11-Tau peptide: MEDHAGTYGLG; (SEQ ID N°9)
Ac-Met1-Tau peptide: {Nα-acetyl}MAEPRQEFEVMEDHAGTYGLG (SEQ ID N°10); the peptide starts at Methionine 1 of Tau harboring an N-alpha-terminal acetylation.

Indirect ELISA screening of hybridoma supernatants allowed selection of a set of clones that specifically detect the Ac-Met11-Tau species with a slight or without any cross-reactivity with the free non-N-alpha-terminally-acetylated Methionine 11, nor with the non-truncated Methionine 11, nor with any N-alpha-acetyl-Methionine when it is not in the same amino acid context than Methionine11. Isotype and the type of light chain have been determined for the selected hybridoma (Table 1, Bellow).

**Table1.**

| Hybridoma designation | Isotype | light chain |
|---|---|---|
| 1C10 | IgG1/IgM | Kappa/Lamda |
| 2H2 | IgG2a | Kappa |
| 3F2 | IgG2A/IgM | Kappa/Lamda |
| 2C12 | IgG1 | Kappa |
| 9H4 | IgG2a | Kappa |

The hybridoma 2H2 was further subcloned and we selected the hybridoma clone that produces 2H2/D11 antibody. The specificity of 2H2/D11 antibody towards N-alpha-terminally acetylated methionine11 of Tau protein was reproducibly validated by indirect ELISA, western blotting and immunohistochemistry. The VH and VL sequences of 2H2D11 are provided.

An antibody against total Tau proteins was also generated following immunization with Met11-Tau peptide: MEDHAGTYGLG; (SEQ ID N°9). We selected the hybridoma clone 7C12/E12 (IgG1, Kappa. The epitope according to the longest human Tau isoform is: aa 11-20). The VH and VL sequences of 7C12/E7) are provided.

Indirect ELISA. Nunc 96-well microtiter plates (Maxisorp F8; Nunc, Inc.) were coated overnight at 4°C with 100ng/well of either Ac-Met11-Tau, Met11-Tau or Ac-Met1-Tau peptide (see above) in 50 mM NaHCO3, pH 9.6. After 3 washes with PBS containing 0.05% Tween (PBS-T), plates were blocked and hybridoma supernatants or purified antibody were tested by use of goat anti-mouse IgG horseradish peroxidase-conjugated antibody (A3673; Sigma) at 1:4000 dilution. Tetramethyl benzidine (T3405, Sigma) was the substrate. The reaction was stopped by addition of sulfuric acid, changing the color from blue to yellow. Plates were measured with a spectrophotometer (Multiskan Ascent Thermo Labsystem) at 450 nm.

Sandwich ELISA. Nunc 96-well plates (VWR) were coated with 100 µl of 2H2/D11 antibody (for detection of Ac-Met11-Tau species) or AT120 (INNOTEST hTau, FUJIREBIO) or 7C12/E7 antibody (for detection of total Tau proteins) at a concentration of 1µg/ml in Carbonate buffer (NaHCO3 0.1M, Na2CO3 0.1M, pH 9.6) overnight at 4°C. The plates were subsequently blocked with a WASH1X buffer (INNOTEST hTau Ag kit, FUJIREBIO) containing 0.1% casein at 37°C for 1 hour and washed with WASH1X buffer 3 times. Protein samples were standardized at 1µg/µl and diluted in SAMPL DIL buffer (INNOTEST hTau Ag kit, FUJIREBIO). Protein samples and biotinylated antibodies (HT7/BT2, INNOTEST hTau Ag kit, FUJIREBIO) were added and the plates were incubated at room temperature overnight. The wells were washed four times then incubated with Peroxidase-labeled streptavidin at room temperature for 30 min and washed four times. Detection was performed using Tetramethyl benzidine substrate for 30 min at room temperature; the assay was stopped with H2SO4 and absorbance was read with spetrophotometer (Multiskan Ascent, Thermo Labsystems) at 450nm.

Tau Plasmid constructs. Expression vectors carrying cDNA for Full-length Tau (Tau-412) and Met11-Tau were generated using the In-Fusion cloning Kit (Clontech), and PCR primers were designed to clone inserts into the EcoRI site of pcDNA4/TO (Invitrogen). Each cDNA fragment was amplified by PCR (DyNAzymeTM EXT DNA polymerase, New England BioLabs) from pcDNA3.1-Tau4R (Mailliot et al., 2000). Forward primers were designed to contain the Kozak consensus sequence and are as follows:
For Tau-412,
   5'-CAGTGTGGTGGAATTCGCCACCATGGCTGAGCCCCGCCAGGAGTT-3'(SEQ ID N°11);
For Met11-Tau,
   5'-CAGTGTGGTGGAATTCGCCACCATGGAAGATCACGCTGGGACGT-3'(SEQ ID N°12);

The reverse primer for the two amplifications was:
5'-GATATCTGCAGAATTCTCACAAACCCTGCTTGGCCAGGG AGGCA-3'(SEQ ID N°13).
DNA sequencing was carried out for construct validation.

Tau cell lines. SH-SY5Y cells that constitutively express tetracycline repressor (detailed described in Hamdane et al., 2003) were transfected with Tau plasmid constructs using ExGen500 (Euromedex, France) according to manufacturer's instructions. Individual stable clones were generated following Zeocin selection (100 µg/ml), and those that exhibited the weakest basal expression of Tau were selected. The cell lines were maintained in Dulbecco's modified Eagle's medium (DMEM, Gibco) supplemented with 10% fetal calf serum with pyruvate, 2mM L-glutamine, 50 units/ml penicillin/streptomycin and 1mM non essential AA. For induction of Tau-412 and Met11-Tau expression, cells were maintained in medium with Tetracycline at 1 µg/ml (Invitrogen) in a 5% CO2 humidified incubator at 37°C.

Protein extractions. Cells were washed using PBS and harvested in ice-cold RIPA buffer: 150mM NaCl, 1% NP40, 0.5% sodium deoxycholate, 0.1% SDS, 50mM Tris-HCl, pH 8.0, completed with protease inhibitors. For humain Brain cortex samples, tissues were homogenized by sonication in a buffer containing 0.32 M sucrose, 100 mM NaCl, 110 mM KOAc, 0.5% Triton X-100, and 10 mM Tris-HCl, pH 7.4 with protease inhibitors (Complete w/o EDTA, Roche), sonicated and centrifuged at 2500 x g for 5 min.

After sonication and homogenization, protein concentrations were determined using the BCA Assay Kit (Pierce).

Western Blotting. Protein extracts were standardized at 1µg/µl with LDS 2X supplemented with a reducing agent (Invitrogen) and denatured at 100°C for 10 min. Proteins were then separated with SDS-PAGE using precast 4-12% Bis-Tris NuPage Novex gels (Invitrogen). Proteins were transferred to 0.45µM nitrocellulose membranes (AmershamTM Hybond ECL), which were saturated with 5% dry non-fat milk in TNT buffer; 140mM NaCl, 0,5% Tween20, 15mM Tris, pH 7.4, or 5% bovine serum albumin (Sigma) in TNT buffer, depending of the primary antibody. Membranes were then incubated with the primary antibody (Table 2, bellow) overnight at 4°C, washed with TNT buffer three times for 10 min, incubated with the secondary antibody (Vector) and washed again before development. Immunolabeling was visualized using chemiluminescence kits (ECLTM, Amersham Bioscience) on an LAS-4000 acquisition system (Fujifilm).

Peptide blocking experiments. Before proceeding to Western Blot or IHC immunostaining, 2H2/D11 antibody was incubated with agitation overnight at 4°C, in blocking buffer without or with excess of the blocking peptide (molar ratio: 1/50): either Ac-Met11-Tau peptide ({Nα-acetyl}MEDHAGTYGLG) or Met11-Tau peptide (MEDHAGTYGLG). Thereafter, the antibody samples were used to perform staining protocol as described in Western Blotting and IHC sections.

Immunohistochemistry (IHC). Thy-Tau22 mice were anesthetized (chloral hydrate 8%) and transcardially perfused with cold PBS followed by 4% paraformaldehyde for 20 min. The brains were removed rapidly, post-fixed overnight in 4% paraformaldehyde, placed in 20% sucrose for 24 hours and finally kept frozen at -80°C until use. Serial free-floating sagittal sections (40µm) were obtained using a cryostat (Leica Microsystems GmbH, Germany). Sections of interest were used for free floating immunohistochemistry. Concerning human brain sections, the hippocampus was dissected, fixed and sliced in the same conditions than mice.

Brain sections were washed with PBS-Triton (0.2%) and treated for 30 min with 0.3% H2O2, and nonspecific binding was blocked with MOM (Mouse IgG blocking reagent) or horse serum (1/100 in PBS; Vector Laboratories) for 1 hour. The sections were then incubated with the primary antibody (Table 2, bellow) in PBS-Triton 0.2% overnight at 4°C. After 3 washes (10 min), labeling was amplified using a biotinylated anti-mouse IgG or rabbit-IgG (1/400 in PBS-Triton 0.2%; Vector Laboratories) for 1 hour, followed by the ABC kit (1/400 in PBS; Vector Laboratories), and labeling was completed using 0.5 mg/ml DAB (Vector Laboratories) in 50 mmol/l Tris-HCl, pH 7.6, containing 0.075% H2O2. Brain sections were mounted on SuperFrost slides, dehydrated through a graded series of alcohol and toluene, and then mounted with Vectamount (Vector Laboratories) for microscopic analysis using Zeiss AXIOSCAN Z1slide scanner and Zen software.

Immunofluorescence (co-labeling experiments). Mice brain sections were washed with PBS-Triton 0.2%, and nonspecific binding was blocked through incubation with MOM (1/100 in PBS-Triton 0,2%; Vector Laboratories) for 1 hour. The sections were then incubated with the first primary antibody in PBS-Triton 0.2% overnight at 4°C. After 3 washes (10 min), the sections were incubated with the second primary antibody in PBS-Triton 0.2% overnight at 4°C. Sections were then washed and incubated with the two secondary antibodies (Invitrogen) coupled to either Alexa 468 or Alexa 588 (1/1000 in PBS) for 1 hour at room temperature. After 3 washes (10 min), the sections were mounted with Vectashield containing DAPI to label the nuclei (Vector Laboratories). Confocal microscopy was performed on a Zeiss LSM 710 inverted confocal microscope (60 magnification). Images were collected in the z direction at 1-µm or 0,8-µm intervals.

**Table 2**

| **Table2** - Summary of primary antibodies used for Western Blotting, Immunohistochemistry and ELISA | | | | |
|---|---|---|---|---|
| Antibody | Species | Dilution | Application(s) | Supplier |
| Tau-Cter (total Tau, 426-441) | rabbit | 1/10000 | WB | Homemade |
| Tau P-Ser422 | rabbit | 1/1000 | IHC | Homemade |
| Tau P-Ser199 | rabbit | 1/500 | IHC | Homemade |
| **2H2/D11 (Ac-Met11-Tau)** | mouse | 1/5000 - 1/200 | WB -ELISA- IHC | Homemade |
| NSE | rabbit | 1/50000 | WB | GeneTex |
| **7C12/E7 (total Tau, 11-21)** | mouse | 1/5000 - 1/200 | WB -ELISA- IHC | Homemade |
| hT7 | mouse | 1/500 | ELISA | Invitrogen |
| Bt2 | mouse | 1/500 | ELISA | Invitrogen |

### Results

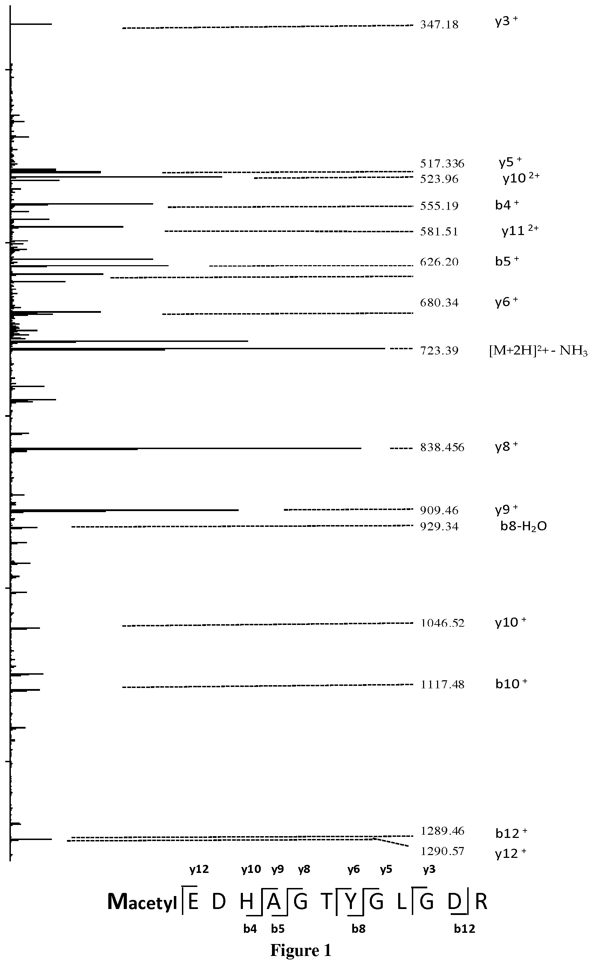

We recently used an optimized proteomics approach and succeeded in identifying new Tau species from the human brain (Derisbourg et al., 2015). Among these latter, the Tau species starting at residue Met11 (Met11-Tau) is also detected as N-alpha-terminally acetylated form (N-alpha-acetyl-Met11-Tau species: Ac-Met11-Tau); such modification has never been described for Tau protein (Fig.1). We expect that these Tau species would be of crucial functional and/or pathological relevance to Alzheimer's disease (AD) and its related Taupathies and would be valuable candidate for diagnosis strategies development. Indeed N-alpha-acetyl-Met11 is located in the exon 1 of Tau protein, which is common to all Tau isoforms. Moreover, it has been shown that any changes in the N-terminal part of Tau could have pathological consequences. For example, mutations of amino acid at position 5 are found associated with Tauopathies (Hayashi et al., 2002 ; Poorkaj et al, 2002.).

The proteomic approach described in Derisbourg et al (2015) allows for the identification but not the quantification of the identified Tau species. Hence, to establish whether Ac-Met11-Tau species is a signature feature of AD and its related Tauopathies and to evaluate the potential of this new Tau species as diagnostic biomarker, we have developed an antibody allowing specific detection of the Ac-Met11-Tau species: 2H2/D11 (Mat&Meth section)(Fig.2). This new antibody does not recognize nor the free non-modified Met11 neither the non-truncated Methionine 11. Also, 2H2/D11 antibody does not recognize N-alpha-acetyl-Methionine when it is not in the same amino acid context than Met11 as it is showed by indirect ELISA using a Tau peptide starting at the Methionine number 1. Isotype strips established that 2H2/D11 is an IgG2a antibody with kappa chain (Table 1, Mat&Meth section).

Thereafter, 2H2/D11 specificity was validated by Western Blotting analysis using protein extracts from cell lines. For this, we generated inducible stable cell lines by transfecting human neuroblastoma cells with tetracycline inducible expression vectors containing the coding sequences of either Full length or Met11 Tau species (FL-Tau (Tau-412) and Met11-Tau, respectively). It should be noted that when we analyzed Met11-Tau cells by the LC-MS/MS proteomic approach, the truncated Met11-Tau protein was found as a mix of non-modified form as well as N-alpha-terminally-acetylated form in this cell line (mass spectra analyses showed a shift of 42 Da corresponding to the addition of an N-alpha-acetyl group to Methionine 11; data not shown).

The transgenes expression by Western Blot, using an antibody against the C-terminal part of Tau that recognizes all Tau species, is displayed in cells treated with tetracycline. Western Blot analysis using 2H2/D11 antibody only labels extracts from the cells expressing the Met11-Tau protein without any cross reactivity with FL-Tau (Fig.3). The specificity of 2H2/D11 antibody towards Ac-Met11-Tau species has been confirmed by experiments where peptide blocking has been performed before proceeding with the immunostaining. As shown in Figure 3, when the antibody is neutralized by the N-alpha-Acetylated-Met11-Tau peptide, the staining is absent, while the immunostaining with the antibody blocked with the Met11-Tau peptide is the same than that with the antibody alone.

After the validation of 2H2/D11 specificity, we used this antibody to establish whether there is an association between Ac-Met11-Tau species and Tau pathology, we used THY-Tau22 mouse model that develops with age neurofibrillary degeneration and memory deficits (For full description, please see Schindowski et al., 2006; Van der Jeugd et al., 2013; Burnouf et al., 2012; 2013). We examined sections of hippocampus from these mice with 2H2/D11 monoclonal antibody. Immunohistochemistry analysis interestingly showed that 2H2/D11 antibody displayed no immunoreactivity with Wt mice while in Thy-Tau22 mice the antibody labels Tau pathology as displayed by characteristic pathological inclusions in neurons. Moreover, this immunolabeling is detected early and seems to increase with age.

The specificity of this finding was further confirmed by experiments where peptide blocking has been performed before proceeding with the immunostaining. When the antibody is neutralized by the N-alpha-Acetylated- Met11-Tau peptide, the staining is absent, while the immunostaining with the antibody blocked with the Met11-Tau peptide is the same than that with the antibody alone.

Furthermore, we performed double labeling experiments using some well-characterized antibodies specific to phosphorylated Tau. We used Phospho-Serine 199 antibody that labels Tau in neurons independently of the presence of neurofibrillary degeneration and Phospho-Serine 422 antibody that only labels pathological Tau. Co-labeling experiments showed that 2H2/D11 antibody detected a subpopulation of neurons that is immunoreactive with the pathological phospho-serine-422 Tau. Overall our data suggest that in mice model, Ac-Met11-Tau protein is found mostly in neurons displaying Tau pathology, and that Ac-Met11 is likely a marker of early stages of pathological process.

We have next examined sections of human brain hippocampus. Immunohistochemistry analysis showed that 2H2D/11 antibody is not reactive in hippocampus from elderly Control. Interestingly, in AD hippocampus the antibody labels characteristic features of neurofibrillary tangles that are the signature of AD.

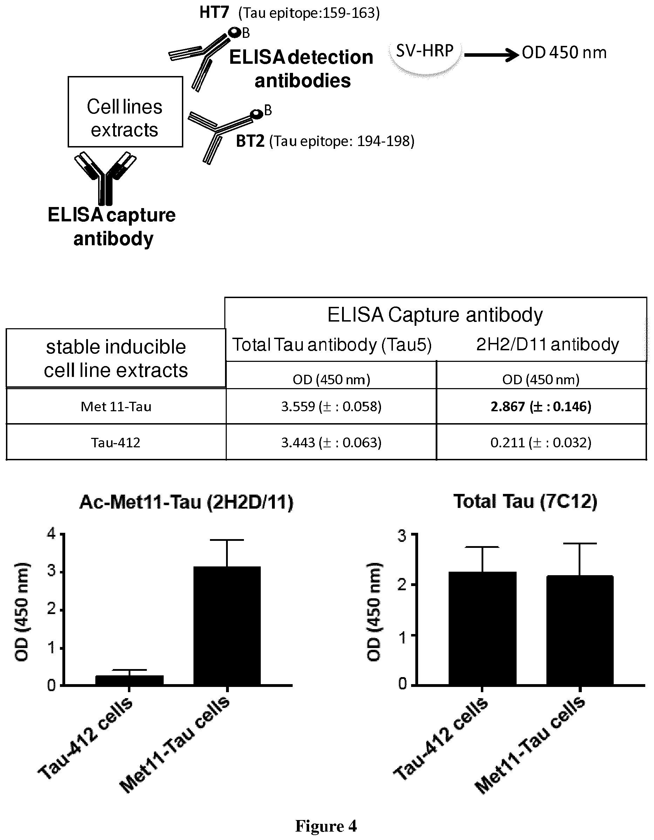

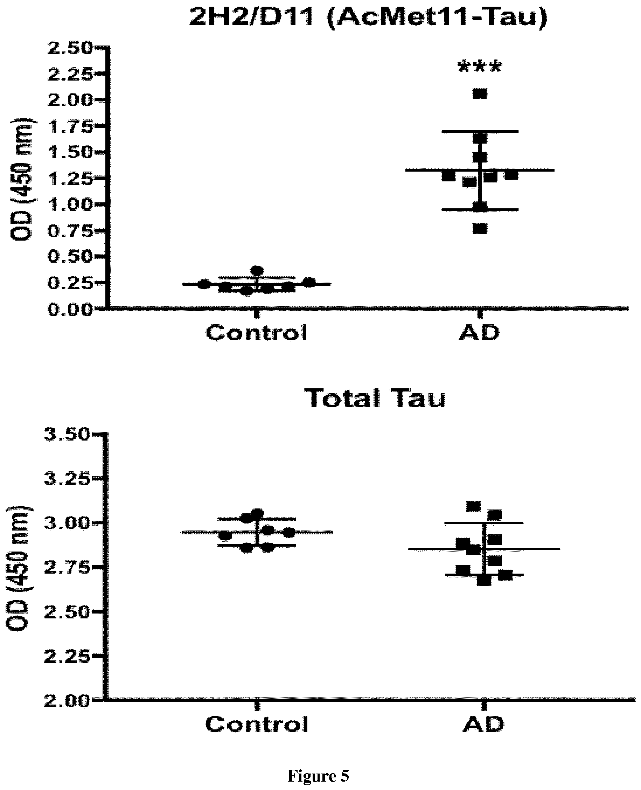

Giving that we attempt to examine whether Ac-Met11-Tau species is potential biomarker for diagnosis, we developed a sandwich ELISA by using protein extracts from stable cell lines (described in Mat & Meth section and Figure 3) to validate 2H2/D11 antibody as a tool to detect and quantify Ac-Met11-Tau species by ELISA. Our data showed that 2H2/D11 antibody works in this immunological application and that our antibody allows specific detection of Ac-Met11-Tau protein (Fig.4). Thereafter, protein extracts from temporal cortex of age-matched controls (n=7) and Alzheimer's disease cases (n=9, from Braak IV-VI) were used in 2H2/D11-based sandwich ELISA. Our data interestingly showed that 2H2/D11 specifically reacted in AD samples ((Fig.5; p=0.0002; compared with Mann-Whitney test). The same data were obtained from Thy-Tau22 transgenic mouse model (not shown).

### Conclusion

We have identified new Tau species starting at methionine 11 and bearing an N-alpha-terminally acetylation. We have developed hybridomas, including the hybridoma that produces 2H2/D11 antibody, allowing specific detection of N-alpha-terminally-acetylated-Met11 Tau species. Our data based on IHC and ELISA assays, from transgenic mice model and human brains, have shown Ac-Met11-Tau specie to be a pathological modification.

Our ongoing studies aim to determine the role of this new Tau specie in AD pathogenesis and to establish whether Ac-Met11-Tau species and its related Met11 species is a signature feature of AD and its related Tauopathies as well as if it is indicative of AD stage. Hence, we are performing detailed immunohistochemistry characterization to further examine this species in AD and other Tauopathies. We will use quantitative and qualitative approaches based on our newly developed reagents in biochemical and histological assays (immunohistochemistry, western Blotting and ELISA) using brains from control subjects, well-characterized AD patients with different staging, and patients with other Tauopathies;. This work will have immediate application for diagnosis.

**Table 3: Useful amino acid sequences for practicing the disclosure**

| **SEQ ID NO** | **Nucleotide or amino acid sequence** |
|---|---|
| 1 : Tau N-Alpha Acetyl-Met11-352 | N-Alpha Acetyl -MEDHAGTYGL GDRKDQGGYT MHQDQEGDTD AGLKAEEAGI GDTPSLEDEA AGHVTQARMV SKSKDGTGSD DKKAKGADGK TKIATPRGAA PPGQKGQANA TRIPAKTPPA PKTPPSSGEP PKSGDRSGYS SPGSPGTPGS RSRTPSLPTP PTREPKKVAV VRTPPKSPSS AKSRLQTAPV PMPDLKNVKS KIGSTENLKH QPGGGKVQIV YKPVDLSKVT SKCGSLGNIH HKPGGGQVEV KSEKLDFKDR VQSKIGSLDN ITHVPGGGNK KIETHKLTFR ENAKAKTDHG AEIVYKSPVV SGDTSPRHLS NVSSTGSIDM VDSPQLATLA DEVSASLAKQ GL |
| 2 : Tau N-Alpha Acetyl-Met11-381 | N-Alpha Acetyl MEDHAGTYGL GDRKDQGGYT MHQDQEGDTD AGLKESPLQT PTEDGSEEPG SETSDAKSTP TAEAEEAGIG DTPSLEDEAA GHVTQARMVS KSKDGTGSDD KKAKGADGKT KIATPRGAAP PGQKGQANAT RIPAKTPPAP KTPPSSGEPP KSGDRSGYSS PGSPGTPGSR SRTPSLPTPP TREPKKVAVV RTPPKSPSSA KSRLQTAPVP MPDLKNVKSK IGSTENLKHQ PGGGKVQIVY KPVDLSKVTS KCGSLGNIHH KPGGGQVEVK SEKLDFKDRV QSKIGSLDNI THVPGGGNKK IETHKLTFRE NAKAKTDHGA EIVYKSPVVS GDTSPRHLSN VSSTGSIDMV DSPQLATLAD EVSASLAKQG L |
| 3 : Tau N-Alpha Acetyl-Met11-383 | N-Alpha Acetyl MEDHAGTYGL GDRKDQGGYT MHQDQEGDTD AGLKAEEAGI GDTPSLEDEA AGHVTQARMV SKSKDGTGSD DKKAKGADGK TKIATPRGAA PPGQKGQANA TRIPAKTPPA PKTPPSSGEP PKSGDRSGYS SPGSPGTPGS RSRTPSLPTP PTREPKKVAV VRTPPKSPSS AKSRLQTAPV PMPDLKNVKS KIGSTENLKH QPGGGKVQII NKKLDLSNVQ SKCGSKDNIK HVPGGGSVQI VYKPVDLSKV TSKCGSLGNI HHKPGGGQVE VKSEKLDFKD RVQSKIGSLD NITHVPGGGN KKIETHKLTF RENAKAKTDH GAEIVYKSPV VSGDTSPRHL SNVSSTGSID MVDSPQLATL ADEVSASLAK QGL |
| 4.Tau N-Alpha Acetyl-Met11-410 | N-Alpha Acetyl MEDHAGTYGL GDRKDQGGYT MHQDQEGDTD AGLKESPLQT PTEDGSEEPG SETSDAKSTP TAEDVTAPLV DEGAPGKQAA AQPHTEIPEG TTAEEAGIGD TPSLEDEAAG HVTQARMVSK SKDGTGSDDK KAKGADGKTK IATPRGAAPP GQKGQANATR IPAKTPPAPK TPPSSGEPPK SGDRSGYSSP GSPGTPGSRS RTPSLPTPPT REPKKVAVVR TPPKSPSSAK |
| | SRLQTAPVPM PDLKNVKSKI GSTENLKHQP GGGKVQIVYK PVDLSKVTSK CGSLGNIHHK PGGGQVEVKS EKLDFKDRVQ SKIGSLDNIT HVPGGGNKKI ETHKLTFREN AKAKTDHGAE IVYKSPVVSG DTSPRHLSNV SSTGSIDMVD SPQLATLADE VSASLAKQGL |
| 5. Tau N-Alpha Acetyl-Met 11-412 | N-Alpha Acetyl - MEDHAGTYGL GDRKDQGGYT MHQDQEGDTD AGLKESPLQT PTEDGSEEPG SETSDAKSTP TAEAEEAGIG DTPSLEDEAA GHVTQARMVS KSKDGTGSDD KKAKGADGKT KIATPRGAAP PGQKGQANAT RIPAKTPPAP KTPPSSGEPP KSGDRSGYSS PGSPGTPGSR SRTPSLPTPP TREPKKVAVV RTPPKSPSSA KSRLQTAPVP MPDLKNVKSK IGSTENLKHQ PGGGKVQIIN KKLDLSNVQS KCGSKDNIKH VPGGGSVQIV YKPVDLSKVT SKCGSLGNIH HKPGGGQVEV KSEKLDFKDR VQSKIGSLDN ITHVPGGGNK KIETHKLTFR ENAKAKTDHG AEIVYKSPVV SGDTSPRHLS NVSSTGSIDM VDSPQLATLA DEVSASLAKQ GL |
| 6 : Tau N-Alpha Acetyl-Met 11-441 (Isoform2) | N-Alpha Acetyl -MEDHAGTYGL GDRKDQGGYT MHQDQEGDTD AGLKESPLQT PTEDGSEEPG SETSDAKSTP TAEDVTAPLV DEGAPGKQAA AQPHTEIPEG TTAEEAGIGD TPSLEDEAAG HVTQARMVSK SKDGTGSDDK KAKGADGKTK IATPRGAAPP GQKGQANATR IPAKTPPAPK TPPSSGEPPK SGDRSGYSSP GSPGTPGSRS RTPSLPTPPT REPKKVAVVR TPPKSPSSAK SRLQTAPVPM PDLKNVKSKI GSTENLKHQP GGGKVQIINK KLDLSNVQSK CGSKDNIKHV PGGGSVQIVY KPVDLSKVTS KCGSLGNIHH KPGGGQVEVK SEKLDFKDRV QSKIGSLDNI THVPGGGNKK IETHKLTFRE NAKAKTDHGA EIVYKSPVVS GDTSPRHLSN VSSTGSIDMV DSPQLATLAD EVSASLAKQG L |
| 7. Tau N-Alpha Acetyl-Met11-776 | N-Alpha Acetyl –MEDHAGTYGL GDRKDQGGYT MHQDQEGDTD AGLKESPLQT PTEDGSEEPG SETSDAKSTP TAEDVTAPLV DEGAPGKQAA AQPHTEIPEG TTAEEAGIGD TPSLEDEAAG HVTQEPESGK VVQEGFLREP GPPGLSHQLM SGMPGAPLLP EGPREATRQP SGTGPEDTEG GRHAPELLKH QLLGDLHQEG PPLKGAGGKE RPGSKEEVDE DRDVDESSPQ DSPPSKASPA QDGRPPQTAA REATSIPGFP AEGAIPLPVD FLSKVSTEIP ASEPDGPSVG RAKGQDAPLE FTFHVEITPN VQKEQAHSEE HLGRAAFPGA PGEGPEARGP SLGEDTKEAD LPEPSEKQPA AAPRGKPVSR VPQLKARMVS KSKDGTGSDD KKAKTSTRSS AKTLKNRPCL SPKHPTPGSS |
| | DPLIQPSSPA VCPEPPSSPK YVSSVTSRTG SSGAKEMKLK GADGKTKIAT PRGAAPPGQK GQANATRIPA KTPPAPKTPP SSATKQVQRR PPPAGPRSER GEPPKSGDRS GYSSPGSPGT PGSRSRTPSL PTPPTREPKK VAVVRTPPKS PSSAKSRLQT APVPMPDLKN VKSKIGSTEN LKHQPGGGKV QIINKKLDLS NVQSKCGSKD NIKHVPGGGS VQIVYKPVDL SKVTSKCGSL GNIHHKPGGG QVEVKSEKLD FKDRVQSKIG SLDNITHVPG GGNKKIETHK LTFRENAKAK TDHGAEIVYK SPVVSGDTSP RHLSNVSSTG SIDMVDSPQL ATLADEVSAS LAKQGL |
| 8. Tau N-Alpha Acetyl-Met 11-21 (antigen) | N-Alpha Acetyl -MEDHAGTYGLG |
| 9. Tau Met1 1-21 | MEDHAGTYGLG |
| 10. Tau N-Alpha Acetyl-Met1-21 | N-Alpha Acetyl -MAEPRQEFEV MEDHAGTYGLG |
| 11. Forward primers for Tau-412 | cagtgtggtggaattcgccaccatggctgagccccgccaggagtt |
| 12. Forward primers for Met11Tau | cagtgtggtggaattcgccaccatggaagatcacgctgggacgt |
| 13. Reverseprimers for both | gatatctgcagaattctcacaaaccctgcttggccaggg |

### EXAMPLE 2:

### Materials and Methods

Indirect ELISA. Nunc 96-well microtiter plates (Maxisorp F8; Nunc, Inc.) were coated overnight at 4°C with 100ng/well of Tau 1-peptide (SEQ ID N°10), Met11-Tau peptide (SEQ ID N°9), or Ac-Met11-Tau peptide (SEQ ID N°8) in 50 mM NaHCO3, pH 9.6. After 3 washes with PBS containing 0.05% Tween (PBS-T), plates were blocked with 0.1% casein solution (PBS) at 37°C for 1 h, followed by incubation with 2H2/D11 antibody, or 7C12/E7 antibody for 1 h at 37°C. Immunodetection was performed by using a goat anti-mouse IgG horseradish peroxidase-conjugated antibody (A3673; Sigma) at 1:4000 dilution. Tetramethyl benzidine (T3405, Sigma) was the substrate. The reaction was stopped by addition of sulfuric acid, changing the color from blue to yellow. Plates were measured with a spectrophotometer (Multiskan Ascent Thermo Labsystem) at 450 nm.

Protein extractions. Human brain autopsy samples (hippocampus) were from the Lille NeuroBank collection (Centre de Ressources Biologiques du CHRU de Lille). Tissues were homogenized by sonication in a buffer containing150mM NaCl, 1% NP40, 0.5% sodium deoxycholate, 0.1% SDS, 50mM Tris-HCl, pH 8.0, completed with protease inhibitors (Complete w/o EDTA, Roche); protein concentrations were determined using the BCA Assay Kit (Pierce).

Sandwich ELISA. Nunc 96-well plates (VWR) were coated with 100 µl of 2H2/D11 antibody (for detection of Ac-Met11-Tau species) or AT120 (INNOTEST hTau, FUJIREBIO) (for detection of total Tau proteins) at a concentration of 1µg/ml in Carbonate buffer (NaHCO3 0.1M, Na2CO3 0.1M, pH 9.6) overnight at 4°C. The plates were subsequently blocked with a WASH1X buffer (INNOTEST hTau Ag kit, FUJIREBIO) containing 0.1% casein at 37°C for 1 hour and washed with WASH1X buffer 3 times. Protein samples were standardized at 1µg/µl and diluted in SAMPL DIL buffer (INNOTEST hTau Ag kit, FUJIREBIO). Protein samples and biotinylated antibodies (HT7/BT2, INNOTEST hTau Ag kit, FUJIREBIO) were added and the plates were incubated at room temperature overnight. The wells were washed four times then incubated with Peroxidase-labeled streptavidin at room temperature for 30 min and washed four times. Detection was performed using Tetramethyl benzidine substrate for 30 min at room temperature; the assay was stopped with H2SO4 and absorbance was read with spetrophotometer (Multiskan Ascent, Thermo Labsystems) at 450nm.

### Results

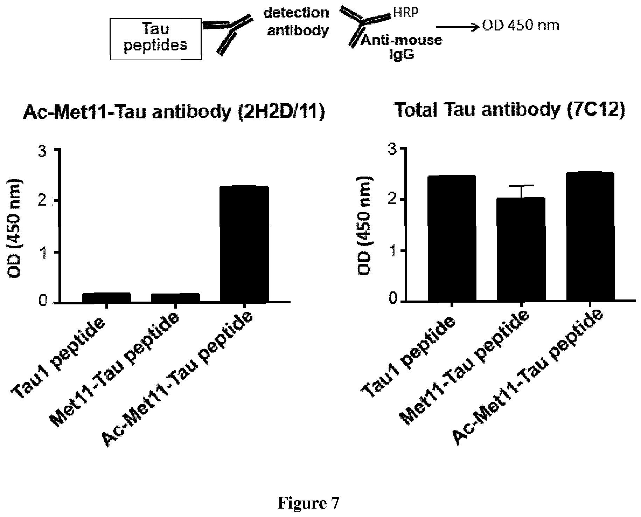

Indirect ELISA reproducibly validated the specificity of 2H2/D11 antibody towards N-alpha-terminally acetylated methionine11 of Tau protein. Indeed, 2H2/D11 displays no reactivity with the free non-N-alpha-terminally-acetylated Methionine 11, or with the non-truncated Methionine 11, or with an N-alpha-acetyl-Methionine when it is not in the same amino acid context than Methionine11. Regarding 7C12/E7 antibody against total Tau proteins, it displays the similar immunoreactivity towards the 3 peptides (figure 7).

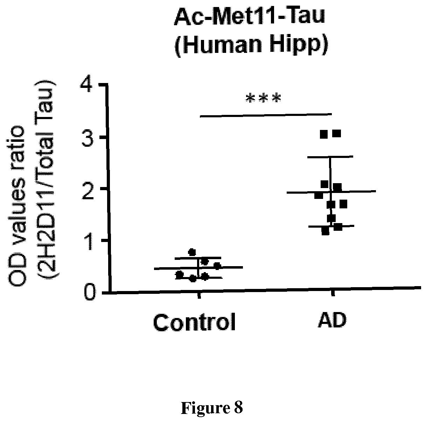

Protein extracts from hippocampus of elderly controls (n=6) and AD cases (n=10, from Braak IV-VI) were used in 2H2D/11-based sandwich ELISA. Data interestingly showed that our antibody specifically reacted in AD hippocampus samples (p=0.0002; compared with Mann-Whitney test) (figure 8).

### REFERENCES:

Throughout this application, various references describe the state of the art to which this disclosure pertains.
Aksnes H, Drazic A, Marie M, Arnesen T. First Things First: Vital Protein Marks by N-Terminal Acetyltransferases. Trends Biochem Sci. 2016;41:746-60.
Arnesen T, Van Damme P, Polevoda B, Helsens K, Evjenth R, Colaert N, Varhaug JE, Vandekerckhove J, Lillehaug JR, Sherman F, Gevaert K. Proteomics analyses reveal the evolutionary conservation and divergence of Nterminal acetyltransferases from yeast and humans. Proc Natl Acad Sci USA. 2009; 106:8157-62.
Barthélemy NR, Gabelle A, Hirtz C, Fenaille F, Sergeant N, Schraen-Maschke S, Vialaret J, Buée L, Junot C, Becher F, Lehmann S. Differential Mass Spectrometry Profiles of Tau Protein in the Cerebrospinal Fluid of Patients with Alzheimer's Disease, Progressive Supranuclear Palsy, and Dementia with Lewy Bodies. J Alzheimers Dis. 2016 Feb 20;51(4):1033-43.
Basurto-Islas, G. et al. Accumulation of aspartic acid421- and glutamic acid391-cleaved tau in neurofibrillary tangles correlates with progression in Alzheimer disease. J Neuropathol Exp Neurol 67, 470-483 (2008).
Braak H, Braak E. Staging of Alzheimer's disease-related neurofibrillary changes. Neurobiol Aging. (1995) 16:271-8.
Braak, H., Thai, D. R., Ghebremedhin, E. & Del Tredici, K. Stages of the pathologic process in Alzheimer disease: age categories from 1 to 100 years. J Neuropathol Exp Neurol 70, 960-969 (2011).
Brandt R, Léger J, Lee G. Interaction of tau with the neural plasma membrane mediated by tau's aminoterminal projection domain. J Cell Biol. 1995; 131:1327-40.
Burnouf S, Belarbi K, Troquier L, Derisbourg M, Demeyer D, Leboucher A, Laurent C, Hamdane M, Buee L, Blum D. Hippocampal BDNF expression in a tau transgenic mouse model. Curr Alzheimer Res. (2012) 9(4):406-10.
Burnouf S, Martire A, Derisbourg M, Laurent C, Belarbi K, Leboucher A, Fernandez-Gomez FJ, Troquier L, Eddarkaoui S, Grosjean ME, Demeyer D, Muhr-Tailleux A, Buisson A, Sergeant N, Hamdane M, Humez S, Popoli P, Buée L, Blum D. NMDA receptor dysfunction contributes to impaired brain-derived neurotrophic factor-induced facilitation of hippocampal synaptic transmission in a Tau transgenic model. Aging Cell. (2013)12:11-23.
Carmel G, Mager EM, Binder LI, Kuret J. The structural basis of monoclonal antibody Alz50's selectivity for Alzheimer's disease pathology. J Biol Chem. 1996; 271:32789-95. 18.
de Calignon, A., Fox, L. M., Pitstick, R., Carlson, G. A., et al. , Caspase activation precedes and leads to tangles. Nature 2010, 464 : 1201-1204.
Delacourte A, David JP, Sergeant N, Buée L, Wattez A, Vermersch P, Ghozali F, Fallet-Bianco C, Pasquier F, Lebert F, Petit H, Di Menza C. The biochemical pathway of neurofibrillary degeneration in aging and Alzheimer's disease. Neurology. (1999) 52:1158-65.
Derisbourg M, Leghay C, Chiappetta G, Fernandez-Gomez FJ, Laurent C, Demeyer D, Carrier S, Buée-Scherrer V, Blum D, Vinh J, Sergeant N, Verdier Y, Buée L, Hamdane M. Role of the Tau N-terminal region in microtubule stabilization revealed by new endogenous truncated forms. Sci Rep. 2015;5:9659.
Drazic A, Myklebust LM, Ree R, Arnesen T. The world of protein acetylation. Biochim Biophys Acta. (2016)1864:1372-401.
Driessen HP, de Jong WW, Tesser GI, Bloemendal H. The mechanism of N-terminal acetylation of proteins. CRC Crit Rev Biochem. 1985;18(4):281-325.
Dujardin S, Lécolle K, Caillierez R, Bégard S, Zommer N, Lachaud C, Carrier S, Dufour N, Aurégan G, Winderickx J, Hantraye P, Déglon N, Colin M, Buée L. Neuron-to-neuron wild-type Tau protein transfer through a trans-synaptic mechanism: relevance to sporadic tauopathies. Acta Neuropathol Commun. 2014; 2:14.
Duyckaerts C, Colle MA, Dessi F, Piette F, Hauw JJ. Progression of Alzheimer histopathological changes. Acta Neurol Belg. 1998; 98:180-5.
Frost B, Jacks RL, Diamond MI. Propagation of tau misfolding from the outside to the inside of a cell. J Biol Chem. 2009; 284:12845-52.
Gamblin TC, Chen F, Zambrano A, Abraha A, Lagalwar S, Guillozet AL, Lu M, Fu Y, Garcia-Sierra F, LaPointe N, Miller R, Berry RW, Binder LI, Cryns VL. Caspase cleavage of tau: linking amyloid and neurofibrillary tangles in Alzheimer's disease. Proc Natl Acad Sci USA. (2003) 100:10032-7.
Goode BL, Feinstein SC. Identification of a novel microtubule binding and assembly domain in the developmentally regulated inter-repeat region of tau. J Cell Biol. 1994;124:769-82.
Guo H, Albrecht S, Bourdeau M, Petzke T, Bergeron C, LeBlanc AC. Active caspase-6 and caspase-6-cleaved tau in neuropil threads, neuritic plaques, and neurofibrillary tangles of Alzheimer's disease. Am J Pathol. (2004) 165:523-31.
Gustke N, Trinczek B, Biernat J, Mandelkow EM, Mandelkow E. Domains of tau protein and interactions with microtubules. Biochemistry. 1994; 33:9511-22.
Hamdane M, Sambo AV, Delobel P, Bégard S, Violleau A, Delacourte A, Bertrand P, Benavides J, Buée L. Mitotic-like tau phosphorylation by p25-Cdk5 kinase complex. J Biol Chem. 2003; 278:34026-34.
Hayashi, S. et al. Late-onset frontotemporal dementia with a novel exon 1 (Arg5His) tau gene mutation. Ann. Neurol. 51, 525-530 (2002).
Hasegawa, M. et al. Protein sequence and mass spectrometric analyses of tau in the Alzheimer's disease brain. J Biol Chem 267, 17047-17054 (1992).
Helsens K, Van Damme P, Degroeve S, Martens L, Arnesen T, Vandekerckhove J, Gevaert K. Bioinformatics analysis of a Saccharomyces cerevisiae N-terminal proteome provides evidence of alternative translation initiation and post-translational N-terminal acetylation. J Proteome Res. 2011;10:3578-89.
Horowitz PM, Patterson KR, Guillozet-Bongaarts AL, Reynolds MR, Carroll CA, Weintraub ST, Bennett DA, Cryns VL, Berry RW, Binder LI. Early N-terminal changes and caspase-6 cleavage of tau in Alzheimer's disease. J Neurosci. (2004) 24:7895-902.
Jeganathan S, von Bergen M, Brutlach H, Steinhoff HJ, Mandelkow E. Global hairpin folding of tau in solution. Biochemistry. 2006;45:2283-93.
Jeganathan S, Hascher A, Chinnathambi S, Biernat J, Mandelkow EM, Mandelkow E. Proline-directed pseudo-phosphorylation at AT8 and PHF1 epitopes induces a compaction of the paperclip folding of Tau and generates a pathological (MC-1) conformation. J Biol Chem. 2008; 283:32066-76.
Johnson GV, Seubert P, Cox TM, Motter R, Brown JP, Galasko D. The tau protein in human cerebrospinal fluid in Alzheimer's disease consists of proteolytically derived fragments. J Neurochem. 1997; 68:430-3.
Jung D, Filliol D, Miehe M, Rendon A. Interaction of brain mitochondria with microtubules reconstituted from brain tubulin and MAP2 or TAU. Cell Motil Cytoskeleton. 1993; 24:245-55.
Kovacech B, Novak M. Tau truncation is a productive posttranslational modification of neurofibrillary degeneration in Alzheimer's disease. Curr Alzheimer Res. 2010;7:708-16.
Lee G, Newman ST, Gard DL, Band H, Panchamoorthy G. Tau interacts with src-family non-receptor tyrosine kinases. J Cell Sci. 1998; 111:3167-77.
Loomis PA, Howard TH, Castleberry RP, Binder LI. Identification of nuclear tau isoforms in human neuroblastoma cells. Proc Natl Acad Sci USA. 1990; 87:8422-6.
Magnani E, Fan J, Gasparini L, Golding M, Williams M, Schiavo G, Goedert M, Amos LA, Spillantini MG. Interaction of tau protein with the dynactin complex. EMBO J. 2007; 26:4546-54.
Mailliot, C. et al. Pathological tau phenotypes. The weight of mutations, polymorphisms, and differential neuronal vulnerabilities. Ann. N. Y. Acad. Sci. 920, 107-114 (2000).
Mandelkow EM, Mandelkow E. Biochemistry and cell biology of tau protein in neurofibrillary degeneration. Cold Spring Harb Perspect Med. 2012; 2:a006247.
McMillan, P. J., Kraemer, B. C., Robinson, L., Leverenz, J. B., et al. , Truncation of tau at E391 promotes early pathologic changes in transgenic mice. J Neuropathol Exp Neurol 2011, 70, 1006-1019.
Mukrasch MD, Biernat J, von Bergen M, Griesinger C, Mandelkow E, Zweckstetter M. Sites of tau important for aggregation populate {beta }-structure and bind to microtubules and polyanions. J Biol Chem. 2005 ;
280:24978-86.
Novak M, Kabat J, Wischik CM. Molecular characterization of the minimal protease resistant tau unit of the Alzheimer's disease paired helical filament. EMBO J. 1993; 12:365-70.
Pandey. Hybridoma technology for production of monoclonal antibodies. Hybridoma (2010) vol. 1 (2) pp. 017.
Polevoda B, Sherman F. Composition and function of the eukaryotic N-terminal acetyltransferase subunits. Biochem Biophys Res Commun. 2003;308:1-11.
Poorkaj, P. et al. An R5L tau mutation in a subject with a progressive supranuclear palsy phenotype. Ann. Neurol. 52, 511-516 (2002).
Rissman RA, Poon WW, Blurton-Jones M, Oddo S, Torp R, Vitek MP, LaFerla FM, Rohn TT, Cotman CW. Caspase-cleavage of tau is an early event in Alzheimer disease tangle pathology. J Clin Invest. (2004) 114:121-30.
Schindowski K, Bretteville A, Leroy K, Bégard S, Brion JP, Hamdane M, Buée L. Alzheimer's disease-like tau neuropathology leads to memory deficits and loss of functional synapses in a novel mutated tau transgenic mouse without any motor deficits. Am J Pathol. (2006) 169:599-616.
Sergeant N, Bretteville A, Hamdane M, Caillet-Boudin ML, Grognet P, Bombois S, Blum D, Delacourte A, Pasquier F, Vanmechelen E, Schraen-Maschke S, Bu6e L. Biochemistry of Tau in Alzheimer's disease and related neurological disorders. Expert Rev Proteomics. (2008) 5:207-24.
Sultan A, Nesslany F, Violet M, Bégard S, Loyens A, Talahari S, Mansuroglu Z, Marzin D, Sergeant N, Humez S, Colin M, Bonnefoy E, Buée L, Galas MC. Nuclear tau, a key player in neuronal DNA protection. J Biol Chem. 2011; 286:4566-75.
Van der Jeugd A, Blum D, Raison S, Eddarkaoui S, Buée L, D'Hooge R. Observations in THY-Tau22 mice that resemble behavioral and psychological signs and symptoms of dementia. Behav Brain Res. (2013) 242:34-9.
von Bergen M, Friedhoff P, Biernat J, Heberle J, Mandelkow EM, Mandelkow E. Assembly of tau protein into Alzheimer paired helical filaments depends on a local sequence motif ((306)VQIVYK(311)) forming beta structure. Proc Natl Acad Sci U S A. 2000; 97:5129-34.
Zhang, Z. et al. Cleavage of tau by asparagine endopeptidase mediates the neurofibrillary pathology in Alzheimer's disease. Nat. Med. (2014). doi:10.1038/nm.3700
Zilka, N. et al. Truncated tau from sporadic Alzheimer's disease suffices to drive neurofibrillary degeneration in vivo. FEBS Letters 580, 3582-3588 (2006).

## Claims

1. An isolated Tau polypeptide which comprises at least 9 consecutive amino acids starting from the methionine residue at position 11 in SEQ ID NO:1-7 wherein said methionine at position 11 is N-alpha acetylated.

2. The isolated polypeptide of Claim 1 which comprises the amino acid sequence starting from the methionine at position 11 to the amino acid at position 776.

3. An isolated polypeptide wherein the polypeptide is selected from the group consisting of:
(i) the amino acids sequence consisting of Tau N-Alpha Acetyl-Met11-352 (SEQ ID N 0:1);
(ii) the amino acids sequence consisting of Tau N-Alpha Acetyl-Met11-381 ( SEQ ID N0:2);
(iii) the amino acids sequence consisting of Tau N-Alpha Acetyl-Met11-383 (SEQ ID N 0:3)
(iv) the amino acids sequence consisting of Tau N-Alpha Acetyl-Met11-410 (SEQ ID N 0:4)
(v) the amino acids sequence consisting of Tau N-Alpha Acetyl-Met11-412 (SEQ ID N0:5);
(vi) the amino acids sequence consisting of Tau N-Alpha Acetyl-Met11-441 (SEQ ID N0:6);
(vii) the amino acids sequence consisting of Tau N-Alpha Acetyl-Met11-776 (SEQ ID N0:7);
(viii) an amino acid sequence at least 80% identical to the sequence of (i) to (vii), said sequence comprises the amino acid sequence starting from the N-Alpha Acetyl Methionine at position 11.
(ix) a fragment of at least 9 consecutive amino acids starting from the N-Alpha Acetyl methionine residue at position 11 of the sequence of (i), to (viii).

4. An isolated polypeptide which comprises or consists of the following amino acid sequence (N-α-acetyl)MEDHAGTYGLG (SEQ ID NO:8).

5. The isolated polypeptide according to Claim 4, of at most 766 amino-acid.

6. The isolated polypeptide according to Claim 4 to 5, for use as an antigen.

7. An antibody that specifically binds to an isolated peptide of any one of claims 1 to 5 wherein the antibody does not bind to a non N-alpha- acetylated form of Methionine 11 Tau polypeptide and/or a N-alpha-acetyl-Metl-Tau polypeptide.

8. The antibody according to claim 7 wherein the antibody is polyclonal or monoclonal.

9. A kit comprising the antibody according to any of claims 7-8.

10. A method for detecting human Tau peptide and/or evaluating its amount in a biological sample, comprising detecting the peptide according to claims 1-5.

11. The method according to claim 10 wherein said method comprises contacting said sample with an antibody according to any of claims 7 to 8.

12. A method according to claims 10 to 11, for the *in vitro* diagnostic of tauopathy.

13. The method according to claim 12, wherein tauopathy is Alzheimer disease.

## Patentansprüche

1. Isoliertes Tau-Polypeptid, das beginnend ab dem Methioninrest an Position 11 in SEQ ID NO: 1-7 mindestens 9 konsekutive Aminosäuren umfasst, wobei das Methionin an Position 11 N-alpha-acetyliert ist.

2. Isoliertes Polypeptid nach Anspruch 1, das beginnend ab dem Methionin an Position 11 bis zu der Aminosäure an Position 776 die Aminosäuresequenz umfasst.

3. Isoliertes Polypeptid, wobei das Polypeptid ausgewählt ist aus der Gruppe, bestehend aus:
(i) der Aminosäurensequenz bestehend aus Tau-N-alpha-acetyl-Met 11-352 (SEQ ID NO:1);
(ii) der Aminosäurensequenz bestehend aus Tau-N-alpha-acetyl-Met11-381 (SEQ ID NO:2);
(iii) der Aminosäurensequenz bestehend aus Tau-N-alpha-acetyl-Met11-383 (SEQ ID NO:3)
(iv) der Aminosäurensequenz bestehend aus Tau-N-alpha-acetyl-Met11-410 (SEQ ID NO:4)
(v) der Aminosäurensequenz bestehend aus Tau-N-alpha-acetyl-Met11-412 (SEQ ID NO:5);
(vi) der Aminosäurensequenz bestehend aus Tau-N-alpha-acetyl-Met11-441 (SEQ ID NO:6);
(vii) der Aminosäurensequenz bestehend aus Tau-N-alpha-acetyl-Met11-776 (SEQ ID NO:7);
(viii) einer Aminosäuresequenz, mindestens 80 % identisch zu der Sequenz von (i) bis (vii), wobei die Sequenz die Aminosäuresequenz beginnend ab dem N-alpha-Acetyl-Methionin an Position 11 umfasst.
(ix) ein Fragment von mindestens 9 konsekutiven Aminosäuren, beginnend ab dem N-alpha-Acetyl-Methioninrest an Position 11 der Sequenz von (i) bis (viii).

4. Isoliertes Polypeptid, das die folgende Aminosäuresequenz (N-α-Acetyl)-MEDHAGTYGLG (SEQ ID NO: 8) umfasst oder daraus besteht.

5. Isoliertes Polypeptid nach Anspruch 4 von höchstens 766 Aminosäure.

6. Isoliertes Polypeptid nach Anspruch 4 bis 5 zur Verwendung als ein Antigen.

7. Antikörper, der spezifisch an ein isoliertes Peptid nach einem der Ansprüche 1 bis 5 bindet, wobei der Antikörper nicht an eine nicht-N-alpha-acetylierte Form von Methionin-11-Tau-Polypeptid und/oder ein N-alpha-Acetyl-Met-1-Tau-Polypeptid bindet.

8. Antikörper nach Anspruch 7, wobei der Antikörper polyklonal oder monoklonal ist.

9. Kit, umfassend den Antikörper nach einem der Ansprüche 7-8.

10. Verfahren zum Nachweisen von humanem Tau-Peptid und/oder Evaluieren seiner Menge in einer biologischen Probe, umfassend Nachweisen des Peptids nach Ansprüchen 1-5.

11. Verfahren nach Anspruch 10, wobei das Verfahren Inkontaktbringen der Probe mit einem Antikörper nach einem der Ansprüche 7 bis 8 umfasst.

12. Verfahren nach Ansprüchen 10 bis 11 zur In-vitro-Diagnose von Tauopathie.

13. Verfahren nach Anspruch 12, wobei Tauopathie Alzheimerkrankheit ist.

## Revendications

1. Polypeptide Tau isolé qui comprend au moins 9 acides aminés consécutifs commençant par le résidu méthionine en position 11 dans SEQ ID NO : 1-7 dans lequel ladite méthionine en position 11 est N-alpha acétylée.

2. Polypeptide isolé selon la revendication 1, qui comprend la séquence d'acides aminés commençant par la méthionine en position 11 jusqu'à l'acide aminé en position 776.

3. Polypeptide isolé dans lequel le polypeptide est choisi dans le groupe consistant en :
(i) la séquence d'acides aminés consistant en Tau N-Alpha-Acétyl-Met11-352 (SEQ ID NO : 1) ;
(ii) la séquence d'acides aminés consistant en Tau N-Alpha-Acétyl-Met11-381 (SEQ ID NO : 2) ;
(iii) la séquence d'acides aminés consistant en Tau N-Alpha-Acétyl-Met11-383 (SEQ ID NO : 3)
(iv) la séquence d'acides aminés consistant en Tau N-Alpha-Acétyl-Met11-410 (SEQ ID NO : 4)
(v) la séquence d'acides aminés consistant en Tau N-Alpha-Acétyl-Met11-412 (SEQ ID NO : 5) ;
(vi) la séquence d'acides aminés consistant en Tau N-Alpha-Acétyl-Met11-441 (SEQ ID NO : 6) ;
(vii) la séquence d'acides aminés consistant en Tau N-Alpha-Acétyl-Met11-776 (SEQ ID NO : 7) ;
(viii) une séquence d'acides aminés au moins 80 % identique à la séquence de (i) à (vii), ladite séquence comprend la séquence d'acides aminés commençant par la N-alpha-acétyl-méthionine en position 11,
(ix) un fragment d'au moins 9 acides aminés consécutifs commençant par le résidu N-alpha-acétyl-méthionine en position 11 de la séquence de (i) à (viii).

4. Polypeptide isolé qui comprend ou consiste en la séquence d'acides aminés suivante (N-α-acétyl)MEDHAGTYGLG (SEQ ID NO : 8).

5. Polypeptide isolé selon la revendication 4, d'au plus 766 acides aminés.

6. Polypeptide isolé selon la revendication 4 à 5, pour utilisant en tant qu'antigène.

7. Anticorps qui se lie spécifiquement à un peptide isolé de l'une quelconque des revendications 1 à 5 dans lequel l'anticorps ne se lie pas à un polypeptide d'une forme non N-alpha-acétylée de Méhtionine 11 Tau et/ou à un polypeptide N-alpha-acétyl-Met1-Tau.

8. Anticorps selon la revendication 7 dans lequel l'anticorps est polyclonal ou monoclonal.

9. Kit comprenant l'anticorps selon l'une quelconque des revendications 7-8.

10. Procédé de détection d'un peptide Tau humain et/ou d'évaluation de sa quantité dans un échantillon biologique, comprenant la détection du peptide selon les revendications 1-5.

11. Procédé selon la revendication 10 dans lequel ledit procédé comprend la mise en contact dudit échantillon avec un anticorps selon l'une quelconque des revendications 7 à 8.

12. Procédé selon les revendications 10 à 11, pour le diagnostic *in vitro* de la tauopathie.

13. Procédé selon la revendication 12, dans lequel la tauopathie est la maladie d'Alzheimer.
